# EUROPÄISCHE PATENTANMELDUNG

(11) **EP 4 765 864 A1**
(43) Veröffentlichungstag der Anmeldung: **24.06.2026**
(21) Anmeldenummer: 25222990.1
(22) Anmeldetag: 12.12.2025
(51) Int. Cl.: H04R 1/10, A61B 5/024

(54) **KOPFHÖRER**

(30) Priorität: 18.12.2024 DE 102024138740; 13.11.2025 DE 102025147004
(71) Anmelder: USound GmbH, 8020 Graz (AT)
(72) Erfinder: RUSCONI CLERICI BELTRAMI, Andrea, 1130 Wien (AT); BOTTONI, Ferruccio, 8020 Graz (AT)
(74) Vertreter: Canzler & Bergmeier Patentanwälte Partnerschaft mbB

(57) **Zusammenfassung**

Die Erfindung betrifft einen Kopfhörer (1), insbesondere On-Ear-Hörer, Over-Ear-Hörer und/oder In-Ear-Hörer, mit zumindest einem Schallwandler (2) zum Erzeugen und Erfassen von zumindest Ultraschallwellen (3), und mit einer Steuereinheit (4), die anhand der erzeugten und erfassten Ultraschallwellen (3) eine Körperfunktion, insbesondere eine Herzfunktion und/oder eine Atemfunktion, eines Trägers (5) des Kopfhörers (1) ermitteln kann. Ferner ist der zumindest eine Schallwandler (2) ein MEMS-Schallwandler (2), insbesondere ein Breitband-MEMS-Schallwandler. Des Weiteren betrifft die Erfindung ein Verfahren zum Ermitteln einer Körperfunktion, insbesondere einer Herzfunktion und/oder Atemfunktion, eines Trägers (5) eines Kopfhörers (1), insbesondere eines On-ear-Hörers, Over-Ear-Hörers und/oder In-Ear-Hörers, bei dem mittels eines MEMS-Schallwandlers (2), insbesondere eines Breitband-MEMS-Schallwandlers, des Kopfhörers (1) Ultraschallwellen (3) erzeugt werden, bei dem mittels des MEMS-Schallwandlers (2), insbesondere des Breitband-MEMS-Schallwandlers, und/oder eines weiteren Mikrofons die Ultraschallwellen (3) erfasst werden und bei dem mittels einer Steuereinheit (4) des Kopfhörers (1) anhand der erzeugten und erfassten Ultraschallwellen (3) die Körperfunktion des Trägers (5) des Kopfhörers (1) ermittelt wird, sowie eine eines, insbesondere breitbandigen, MEMS-Schallwandlers (2) für einen Kopfhörer (1) und/oder ein Verfahren zum Ermitteln einer Körperfunktion.

## Beschreibung

Die vorliegende Erfindung betrifft einen Kopfhörer, insbesondere On-Ear-Hörer, Over-Ear-Hörer und/oder In-Ear-Hörer, mit zumindest einem Schallwandler zum Erzeugen und Erfassen von zumindest Ultraschallwellen, und mit einer Steuereinheit, die anhand der erzeugten und erfassten Ultraschallwellen zumindest ein Merkmal, insbesondere eine Körperfunktion, vorzugsweise eine Herzfunktion und/oder eine Atemfunktion, und/oder eine Identität, eines Trägers des Kopfhörers ermitteln und/oder eine Authentifikation des Trägers durchführen kann.

Aufgabe der vorliegenden Erfindung ist es, einen Kopfhörer bereitzustellen, mit dem Merkmale des Trägers zuverlässig, mit hoher Messqualität und/oder schonend für das Ohr ermittelt werden können.

Die Aufgabe wird gelöst durch einen Kopfhörer, ein Verfahren und/oder die Verwendung eines MEMS-Schallwandlers mit den Merkmalen der unabhängigen Ansprüche. Vorteilhafte oder bevorzugte Ausführungen sind jeweils Gegenstand eines korrespondierenden abhängigen Anspruchs.

Vorgeschlagen wird ein Kopfhörer. Der Kopfhörer kann hierbei ein On-Ear-Hörer, ein O-ver-Ear-Hörer und/oder ein In-Ear-Hörer sein. On-Ear- bzw. Over-Ear-Hörer werden hierbei über den Ohren getragen. Bei den In-Ear-Hörern handelt es sich um Kopfhörer, die zumindest teilweise in den Gehörgang eines Benutzers bzw. Trägers eingeführt sind.

Der Kopfhörer umfasst zumindest einen Schallwandler zum Erzeugen und Erfassen von zumindest Ultraschallwellen.

Ferner umfasst der Kopfhörer eine Steuereinheit, die anhand der erzeugten und erfassten Ultraschallwellen zumindest ein Merkmal, insbesondere eine Körperfunktion, vorzugsweise eine Herzfunktion und/oder eine Atemfunktion, und/oder eine Identität, eines Trägers des Kopfhörers ermitteln und/oder eine Authentifikation des Trägers durchführen kann. Der Kopfhörer kann somit dazu dienen, Vitalfunktionen des Trägers, wie beispielsweise dessen Herzfrequenz und/oder Atemfrequenz, zu ermitteln. Zusätzlich oder alternativ kann mit Hilfe des Kopfhörers der Träger identifiziert werden. Im Folgenden kann die Erfindung überwiegend anhand der Messung der Körperfunktion erklärt sein. Zusätzlich oder alternativ kann auch die Identität des Trägers erfasst werden. Zusätzlich oder alternativ kann auch die Authentifikation des Trägers durchgeführt werden.

Der zumindest eine Schallwandler ist ein MEMS-Schallwandler, der zumindest Ultraschallwellen in einem Breitbandspektrum erzeugen kann. Der MEMS-Schallwandler kann ein breitbandiger MEMS-Schallwandler sein. Zusätzlich oder alternativ kann der MEMS-Schallwandler ein Breitband-MEMS-Schallwandler sein. Zusätzlich oder alternativ kann der MEMS-Schallwandler ein MEMS-Breitband-Schallwandler sein. Zusätzlich oder alternativ kann der MEMS-Schallwandler ein MEMS-Breitband-Ultraschallwandler sein. Zusätzlich oder alternativ kann der MEMS-Schallwandler ein MEMS-Breitband-Ultraschalllautsprecher sein. Im Folgenden kann der Einfachheit halber lediglich von einem MEMS-Schallwandler gesprochen werden, auch wenn er ein MEMS-Breitbandschallwandler, ein MEMS-Breitbandultraschallwandler und/oder ein MEMS-Breitbandultraschalllautsprecher usw. ist.

Da mittels des MEMS-Schallwandlers das Breitbandspektrum erzeugt werden kann, stehen eine Vielzahl an möglichen Ultraschallwellen mit verschiedenen Frequenzen bzw. Messfrequenzen zur Messung der Körperfunktion zur Verfügung. Der MEMS-Schallwandler hat hierbei die Fähigkeit verschiedene Ultraschallwellen mit verschiedenen Frequenzen des Breitbandspektrums zu erzeugen, so dass verschiedene Messfrequenzen der Ultraschallwellen zur Verfügung stehen. Beim Messen der Körperfunktion werden hierbei die Ultraschallwellen ausgesendet, die dann mit dem Gewebe, dem Ohr, dem Ohrkanal usw. wechselwirken. Die dadurch veränderten Ultraschallwellen bzw. die reflektierten Ultraschallwellen werden wieder erfasst und ausgewertet, woraus die Körperfunktion ermittelt wird. Der MEMS-Schallwandler bietet die Möglichkeit, mit vielfältigen Ultraschallfrequenzen die Körperfunktion zu messen. Der MEMS-Schallwandler kann über das ganze Breitbandspektrum qualitativ hochwertige Ultraschallwellen zur Messung der Körperfunktion erzeugen.

Durch die Verwendung des MEMS-Schallwandlers, der die Ultraschallwellen über das Breitbandspektrum erzeugen kann, d.h. mit Hilfe eines Breitband-MEMS-Schallwandlers, kann eine verbesserte Auflösung, Genauigkeit bei der Messung und/oder qualitativ hochwertige und/oder zuverlässige Messung der Körperfunktion erzielt werden. Durch die Fähigkeit der Erzeugung der Ultraschallwellen über das Breitbandspektrum, d.h. die Möglichkeit, dass der MEMS-Schallwandler Ultraschallwellen mit verschiedenen Frequenzen aus dem Breitbandspektrum erzeugen kann, kann die Messfrequenz der ausgesendeten Ultraschallwellen individuell an den Gehörgang bzw. das Ohr bzw. an den Träger angepasst werden. Da sich die Gehörgänge und die Ohren von verschiedenen Trägern unterscheiden, kann es für eine zuverlässige Messung der Körperfunktion vorteilhaft sein, eine andere Frequenz, d.h. eine an den speziellen Gehörgang, das Ohr bzw. den Träger angepasste Frequenz der Ultraschallwellen, zu wählen. Welche Frequenz der Ultraschallwellen zum Messen der Körperfunktion verwendet wird, hängt auch davon ab, wie und wo der Kopfhörer getragen wird. Beispielsweise können beim On-Ear-Hörer bzw. Over-Ear-Hörer andere Frequenzen vorteilhafter sein als beim In-Ear-Hörer.

Mit Hilfe des MEMS-Schallwandlers, der die Ultraschallwellen aus dem Breitbandspektrum erzeugen kann, kann die Messung der Körperfunktion auch für verschiedene Träger mit verschiedenen Gehörgängen bzw. Ohren zuverlässig durchgeführt werden. Der MEMS-Schallwandler kann hierbei die Ultraschallwellen aus dem Breitbandspektrum und über das Breitbandspektrum mit einer hohen Qualität erzeugen. Der MEMS-Schallwandler kann eine Vielzahl an Frequenzen bzw. alle Frequenzen aus dem Breitbandspektrum bereitstellen, die zum Messen der individuellen Körperfunktion verwendet werden können.

Vorteilhaft ist es, wenn der MEMS-Lautsprecher das Breitbandspektrum mit einem hörbaren Wellenlängenspektrum und einem Ultraschallspektrum erzeugen kann, so dass als Schallwellen hörbare Schallwellen und Ultraschallwellen erzeugt werden können. Diese vom MEMS-Schallwandler erzeugbaren Schallwellen aus dem Breitbandspektrum können in einem Frequenzbereich von größer gleich 3 kHz, 4 kHz oder 15 kHz bis kleiner gleich 40 kHz, 80 kHz oder 100 kHz liegen. Ein Breitbandlautsprecher, der Schall im hörbaren Bereich und im Ultraschallbereich erzeugen kann, bietet den Vorteil einer breiteren Frequenzabdeckung. Diese Frequenzvielfalt, von beispielsweise 3 kHz bis 100 kHz, ermöglicht zum einen die Erzeugung von hörbaren Schallwellen, so dass mit dem Kopfhörer beispielsweise Musik gehört werden kann. Zum anderen können durch den MEMS-Schallwandler die Ultraschallwellen aus dem Breitbandspektrum, beispielsweise bis hoch zu 100 kHz, zur Messung der Körperfunktion erzeugt werden. Des Weiteren kann hierdurch der Informationsgehalt bei der Messung erhöht werden, da ein großer Frequenzbereich zur Verfügung steht. Es besteht die Möglichkeit Ultraschallwellen mit verschiedenen Messfrequenzen aus dem Breitbandspektrum zu erzeugen, mit denen verschiedene Messungen durchgeführt werden können.

Des Weiteren ist es vorteilhaft, wenn die Steuereinheit und/oder der MEMS-Lautsprecher derart ausgebildet und/oder eingerichtet sind, dass zur Ermittlung der Körperfunktion ein Messsignal aus Ultraschallwellen mit zumindest einer Messfrequenz aus dem Breitbandspektrum erzeugbar ist. Mit Hilfe des Messsignals kann die Messung der Körperfunktion durchgeführt werden. Das Messsignal wird ausgesendet, interagiert mit dem Ohr, beispielsweise dem Gewebe und/oder dem Gehörgang, und gelangt als reflektierte Ultraschallwellen zurück, wobei die reflektierten Ultraschallwellen erfasst werden. Anhand eines Vergleichs zwischen dem ausgesendeten Messsignal und den erfassten, reflektierten Ultraschallwellen kann die Körperfunktion ermittelt werden. Das Messsignal kann hierbei eine oder mehrere Messfrequenzen aufweisen, die aus dem durch den MEMS-Schallwandler erzeugbaren Breitbandspektrum stammen.

Vorteilhaft ist es, wenn die Steuereinheit und/oder der MEMS-Lautsprecher derart ausgebildet und/oder eingerichtet sind, dass die vom MEMS-Schallwandler erzeugten Ultraschallwellen des Messsignals ein oder mehrere, insbesondere diskrete, Messfrequenzen umfassen. Das Breitbandspektrum weist hierbei einen oder mehrere Peaks bzw. Messfrequenzpeaks auf, die durch das eine oder die mehreren Messsignale gegeben sind. Die Messsignale sind durch einzelne Frequenzen charakterisiert, die im Breitbandspektrum sehr schmal sind. Durch die klar definierten Peaks der Messsignale kann die Auswertung erleichtert werden, da die reflektierten Ultraschallwellen einfach mit den Messsignalen und mit den Messfrequenzpeaks verglichen werden können. Des Weiteren kann dadurch ein hohes Signal-Rauschen-Verhältnis erzielt werden und unerwünschte Störfrequenzen können leichter ausgeblendet werden, was zu einer höheren Messgenauigkeit führt.

Vorteilhaft ist es ferner, wenn die Ultraschallwellen des Messsignals kontinuierliche Messfrequenzen umfassen, so dass das Messsignal im Breitbandspektrum ein breites Messfrequenzspektrum aufweist. Ein breites Messfrequenzspektrum bietet den Vorteil, dass eine größere Menge an Informationen über die reflektierten Signale gesammelt werden kann. Dies führt zu einer detaillierteren Analyse. Außerdem ermöglicht ein kontinuierliches und breites Messfrequenzspektrum, dass damit die Körperfunktionen eines breiten Spektrums verschiedener Träger gemessen werden können. Eine Anpassung der Messfrequenz an eine spezielle Ohrform entfällt hierbei.

Die diskreten Messfrequenzen, d.h. das Messsignal mit zumindest einem Messfrequenzpeak, können vorteilhaft sein, um einen gezielten / bestimmten Signalbereich für die Analyse zu isolieren und damit die Erkennung von Mustern oder Veränderungen im Signal zu verbessern. Durch das kontinuierliche Messfrequenzspektrum ist eine umfassende und informationsreiche Datenerfassung möglich, um komplexe Reflexionen und Interaktionen genauer zu analysieren.

Der Vorteil der diskreten Messfrequenzen besteht darin, dass scharfe Peaks im Frequenzbereich die Auswertung erleichtern. Der Vorteil des kontinuierlichen Spektrums besteht darin, dass es eine detaillierte Analyse komplexer Signale ermöglicht, indem es die größeren Bereiche des Breitbandspektrums ausnutzt.

Vorteilhaft ist es, wenn die Steuereinheit und/oder der MEMS-Lautsprecher derart ausgebildet und/oder eingerichtet sind, dass der zumindest eine Messfrequenzpeak des Messsignals eine Breite von weniger als 3 kHz, 2 kHz oder 1 kHz aufweist. Durch die geringe Breite des Messfrequenzpeaks wird eine präzise Frequenzselektion ermöglicht, was die Unterdrückung und/oder das Erkennen von Störungen und unerwünschten Signalanteilen erleichtert. Dies führt zu einer höheren Genauigkeit bei der Analyse und verbessert die Signalqualität insgesamt, da das relevante Frequenzband klarer und definierter erfasst wird. Das Messsignal ist hierbei sehr schmalbandig, stammt jedoch aus dem Breitbandspektrum. Das Messsignal mit den Messfrequenzpeaks kann beispielsweise zwischen 3 kHz und 100 kHz liegen. Beispielsweise kann ein Messsignal vom MEMS-Schallwandler mit einem Messfrequenzpeak bei 30 kHz und einer Breite von 2 kHz erzeugt werden.

Eine schmalbandige Auslegung des Messfrequenzpeaks optimiert die Sensitivität für feine Änderungen und/oder Muster im Zielsignal bzw. bei den reflektierten Ultraschallwellen. Gleichzeitig reduziert diese Fokussierung den Aufwand bei der Signalverarbeitung, da weniger irrelevante Daten berücksichtigt werden müssen, was die Fehleranfälligkeit verringert und die Messung zuverlässiger macht.

Darüber hinaus ist es vorteilhaft, wenn die Steuereinheit und/oder der MEMS-Schallwandler derart ausgebildet und/oder eingerichtet sind, dass das Messfrequenzspektrum des Messsignals eine Frequenzbreite von größer gleich 1 kHz oder größer gleich 5 kHz oder größer gleich 10 kHz oder größer gleich 20 kHz oder größer gleich 30 kHz oder größer gleich 40 kHz oder größer gleich 50 kHz oder größer gleich 60 kHz oder größer gleich 70 kHz oder größer gleich 80 kHz aufweist. Zusätzlich oder alternativ kann das Messfrequenzspektrum des Messsignals eine Frequenzbreite von kleiner als 70 kHz oder von kleiner als 60 kHz oder von kleiner als 50 kHz oder von kleiner als 40 kHz oder von kleiner als 30 kHz oder von kleiner als 20 kHz oder von kleiner als 10 kHz aufweisen. Hierbei ist natürlich die obere Grenze stets größer als die untere Grenze. Beispielsweise kann das Messfrequenzspektrum des Messsignals eine Frequenzbreite von 30 kHz bis 55 kHz aufweisen. Die Frequenzbreite beträgt hier 25 kHz. Mit Hilfe einer großen Frequenzbreite können des Weiteren mehr Informationen gesammelt bzw. gemessen werden, da mehr Frequenzen zur Messung zur Verfügung stehen.

Vorteilhafterweise ist der MEMS-Schallwandler derart ausgebildet und/oder eingerichtet, dass die im Breitbandspektrum erzeugbaren Schallwellen einen Schalldruckpegel von größer gleich 50 dB, bevorzugt größer gleich 55 dB, besonders bevorzugt größer gleich 70 dB aufweisen. Des Weiteren sind diese Schalldruckpegel schonend für das Ohr und/oder das Gewebe.

Gemäß einer vorteilhaften Weiterbildung der Erfindung ist der MEMS-Schallwandler derart ausgebildet und/oder eingerichtet, dass die im Breitbandspektrum erzeugbaren Schallwellen einen Schalldruckpegel von kleiner gleich 90 dB, bevorzugt kleiner gleich 85 dB, besonders bevorzugt kleiner gleich 80 dB, aufweisen. Die Begrenzung des Schalldruckpegels reduziert die Belastung des Gehörs und erhöht den Komfort für den Träger bei der kontinuierlichen Überwachung. Dies ist besonders wichtig für eine langfristige Anwendung. Durch diesen Schalldruckpegel wird das Ohr und/oder das Gewebe nicht geschädigt.

Des Weiteren ist es vorteilhaft, wenn der MEMS-Schallwandler derart ausgebildet und/oder eingerichtet ist, dass die im Breitbandspektrum erzeugbaren Schallwellen einen Schalldruckpegel, insbesondere über das gesamte Breitbandspektrum, mit einer Abweichung von +/- 10 dB, insbesondere +/- 5 dB, um einen Mittelwert aufweisen. Eine geringe Abweichung des Schalldruckpegels gewährleistet eine hohe Präzision der Messung, was die Zuverlässigkeit der Körperfunktionsüberwachung verbessert. Insbesondere das Messfrequenzspektrum des Messsignals kann über dessen gesamte Frequenzbreite diese Abweichung aufweisen.

Darüber hinaus ist es vorteilhaft, wenn der MEMS-Schallwandler derart ausgebildet und/oder eingerichtet ist, dass die im Breitbandspektrum erzeugbaren Schallwellen, insbesondere über das gesamte Breitbandspektrum, ein kontinuierliches Spektrum aufweisen. Der MEMS-Schallwandler kann somit alle Frequenzen im Breitbandspektrum, beispielsweise von 3 kHz bis 100 kHz, ausbilden, so dass zum einen hörbare Schallwellen erzeugt werden können. Zum anderen stehen damit alle Ultraschallwellen, beispielsweise bis 100 kHz, zum Messen der Körperfunktionen zur Verfügung. Infolgedessen stehen mehrere Frequenzen für die Messung zur Verfügung.

Vorteilhaft ist es, wenn der Kopfhörer und/oder die Steuereinheit einen Modulator umfasst, der ein elektrisches Signal, das den hörbaren Schallwellen entspricht, und ein elektrisches Signal, das dem Messsignal im Ultraschallbereich entspricht, zusammenfassen und/oder modulieren kann. Dadurch wird es möglich, beide Signalarten effizient über denselben MEMS-Schallwandler auszugeben, der aus dem modulierten Signal sowohl Schallwellen im hörbaren Frequenzbereich als auch im Ultraschallbereich erzeugt. Hierdurch können die hörbaren Schallwellen und die Ultraschallwellen gleichzeitig und/oder simultan vom MEMS-Schallwandler erzeugt werden. Diese Integration reduziert die Komplexität des Systems, da keine separaten Schallwandler für die beiden Frequenzbereiche erforderlich sind. Gleichzeitig wird der Platzbedarf minimiert, was besonders bei kompakten Geräten wie Kopfhörern von Vorteil ist. Zudem ermöglicht die Modulation eine präzise Steuerung der Signalzusammensetzung, wodurch die Qualität und Effizienz der erzeugten Schallwellen verbessert werden. Dies bietet eine vielseitige Nutzbarkeit in unterschiedlichen Anwendungen, wie der Audioausgabe und der Vitalüberwachung.

Vorteilhaft ist es, wenn der Kopfhörer und/oder die Steuereinheit einen Signalgenerator umfasst, der zeitdiskrete Pulse, Chirp-, Sweep- und/oder Multiton-Signale zum Erzeugen der Ultraschallwellen zum Ermitteln der Körperfunktion erzeugen kann. Die Nutzung von Chirp- und Sweepsignalen ermöglicht eine effektive Analyse der Gewebereflexionen und verbessert die Messgenauigkeit durch eine größere Frequenzabdeckung. Mit Hilfe dieser Signale kann der Ultraschall, insbesondere mit dem kontinuierlichen Spektrum, erzeugt werden.

Von Vorteil ist es, wenn der Kopfhörer einen MLS-Signalerzeuger umfasst, mittels dem ein Maximum-Length-Sequence-Signal erzeugt werden kann, auf Basis dessen der MEMS-Schallwandler zumindest die Ultraschallwellen im Breitbandspektrum erzeugen kann. Von Vorteil ist es, wenn der Signalgenerator den MLS-Signalerzeuger umfasst oder der MLS-Signalerzeuger ist. Der Signalgenerator kann somit zusätzlich oder alternativ zu den zeitdiskreten Pulse, Chirp-, Sweep- und/oder Multiton-Signalen zum Erzeugen der Ultraschallwellen auch das Maximum-Length-Sequence-Signal erzeugen.

Zur Durchführung der Messung bzw. zum Durchführen des Verfahrens mittels des Kopfhörers kann das sogenannte Maximum-Length-Sequence-Signal (MLS) verwendet werden. Unter einem MLS-Signal wird eine pseudozufällige Bitfolge verstanden, deren spektrale Energiedichte über einen breiten Frequenzbereich gleichmäßig verteilt ist und deren Autokorrelation einem schmalen Impuls entspricht. Dadurch können alle Frequenzen im Frequenzbereich gleichzeitig angeregt werden, was eine vollständige Bestimmung der akustischen Impulsantwort des Gehörgangs ermöglicht. Der technische Vorteil liegt in der gleichmäßigen spektralen Anregung und in der Möglichkeit, das Übertragungsverhalten des Ohrs ohne vorherige Kalibrierung zu bestimmen.

Das Verfahren sieht vor, dass die aus dem MLS-Signal erzeugten Ultraschallwellen durch die Schallwandlereinheit, beispielsweise durch die MEMS-Schallwandlereinheit, in den Gehörgang abgestrahlt werden. Das im Ohr reflektierte Echosignal bzw. die reflektierten Ultraschallwellen kann von derselben oder einer weiteren Schallwandlereinheit aufgenommen werden. Durch die Auswertung der Laufzeit- und Amplitudenverhältnisse zwischen Sendesignal und Echosignal können Merkmale des Benutzers bestimmt werden.

Das MLS-Signal wird in digitaler Form erzeugt und anschließend gefiltert. Die Filterung dient dazu, den Frequenzbereich des Signals an das jeweilige Messszenario anzupassen, insbesondere indem hörbare Frequenzen entfernt und der nutzbare Bereich in den Ultraschallbereich verschoben werden. Die resultierende Signalform weist eine gleichmäßige Energieverteilung über die gewünschten Frequenzen auf, wodurch Intermodulationsverzerrungen breitbandig verteilt und somit messtechnisch unkritisch werden.

Nach der Filterung wird das Signal durch einen Digital-Analog-Wandler in ein elektrisches Sendesignal überführt und an die Schallwandlereinheit ausgegeben. Das durch Reflexion und Absorption im Ohr veränderte akustische Echosignal wird über einen Analog-Digital-Wandler digitalisiert und einer Verarbeitungseinheit zugeführt. Durch eine Kreuzkorrelation zwischen Sende- und Empfangssignal kann die Impulsantwort des Systems bestimmt werden. Diese enthält sämtliche akustischen Eigenschaften des Gehörgangs und bildet die Grundlage für die Ermittlung physiologischer Parameter.

Eine Verarbeitungseinheit und/oder die Steuereinheit kann zusätzlich spektrale Analysen, wie Fourier-, Wavelet- oder Periodogramm-Analysen, durchführen, um zeitlich veränderliche Eigenschaften der Impulsantwort oder des Frequenzgangs zu bestimmen. Zusätzlich oder alternativ kann auch eine Cepstrumanalyse von der Verarbeitungseinheit und/oder der Steuereinheit durchgeführt werden. Auf diese Weise lassen sich kleinste Änderungen im akustischen Verhalten des Ohrs erfassen, die beispielsweise durch Pulsation, Atmung oder Kopfbewegungen verursacht werden. Mit Hilfe der Cepstrumanalyse kann beispielsweise der Träger besonders gut erkannt werden.

Die hier erwähnte Verarbeitungseinheit kann die Steuereinheit sein. Alternativ kann die Steuereinheit die Verarbeitungseinheit umfassen.

Ein besonderer Vorteil der Erfindung liegt darin, dass die Messung unabhängig von der geometrischen Form des Gehörgangs, der genauen Position des Kopfhörers und einer vorherigen Kalibrierung auf eine spezielle, dem Träger zugeordnete Messfrequenz durchgeführt werden kann. Da das MLS-Signal alle Frequenzen im Frequenzbereich gleichzeitig anregt, werden anatomische Unterschiede automatisch berücksichtigt, wodurch keine individuelle Kalibrierung erforderlich ist.

Durch den Einsatz einer einzigen Schallwandlereinheit, die abwechselnd als Lautsprecher und Mikrofon betrieben wird, kann die Baugröße des Systems reduziert und eine hohe Messgenauigkeit erreicht werden. Gleichzeitig ermöglicht die Integration in handelsübliche Kopfhörer eine unauffällige und dauerhafte Anwendung. Die Erfindung ermöglicht somit ein robustes, breitbandiges und kalibrierfreies Messverfahren zur Bestimmung individueller oder physiologischer Merkmale eines Benutzers unter Verwendung akustischer Signale im Ultraschallbereich.

Von Vorteil ist es, wenn das Maximum-Length-Sequence-Signal mittels eines MLS-Signalerzeugers erzeugt wird. Ein MLS-Signalerzeuger implementiert typischerweise ein lineares Rückkopplungsschieberegister (LFSR), das eine definierte Bitfolge mit maximaler Periodenlänge generiert. Dadurch ist die Signalform exakt reproduzierbar, was eine präzise Referenz für die Kreuzkorrelation mit dem Echosignal schafft.

Es ist vorteilhaft, wenn das Maximum-Length-Sequence-Signal mittels eines linearen Feedback-Shift-Registers mit einer Periodenlänge von 2^n-1 erzeugt wird, wobei die Bitzahl n des Schieberegisters in einem Bereich von 8 bis 16 liegt. Ein größerer n-Wert führt zu feinerer Frequenzauflösung und höherem Signal-Rausch-Verhältnis.

Vorteilhafterweise wird erzeugter Ultraschall mit einer Abtastrate zwischen 50 kHz und 200 kHz, insbesondere mit 96 kHz, erzeugt und/oder verarbeitet. Die Abtastrate legt die höchste messbare Frequenz (Nyquist-Grenze) und die zeitliche Auflösung der Messung fest. Durch 96 kHz wird der Ultraschallbereich bis 48 kHz abgedeckt, was eine präzise Erfassung der Ohrakustik über den Hörbereich hinaus erlaubt.

In einer vorteilhaften Weiterbildung der Erfindung wird das durch den MLS-Signalerzeuger erzeugte Maximum-Length-Sequence-Signal von zumindest einer Filtereinheit gefiltert. Dadurch entsteht ein digitales Sendesignal, welches den Ultraschall erzeugt. Unter Filterung wird die frequenzselektive Anpassung des Signals verstanden, um unerwünschte Frequenzen zu unterdrücken. Durch digitale Filterung können hörbare Frequenzen entfernt und die spektrale Form des Signals an den Messzweck angepasst werden. Es wird somit ein akustisches Sendesignal, also der Ultraschall, erzeugt, welches vollständig im Ultraschallbereich liegt, so dass der Benutzer durch die Messung nicht gestört wird.

Es ist vorteilhaft, wenn die zumindest eine Filtereinheit eine Bandbreitenlimitierung durchführt, wobei insbesondere Frequenzen im hörbaren Bereich entfernt werden. Dies verhindert akustische Wahrnehmbarkeit des Messsignals durch den Benutzer und reduziert Störinterferenzen mit Wiedergabesignalen.

Darüber hinaus ist es vorteilhaft, wenn die zumindest eine Filtereinheit eine Vorverzerrung durchführt. Eine Vorverzerrung kompensiert die frequenzabhängige Empfindlichkeit von Lautsprecher und Mikrofon. Dadurch wird die Gesamtübertragungsfunktion linearisiert, was die Qualität der rekonstruierten Impulsantwort erhöht.

Die Filtereinheit ist vorzugsweise als digitale Filtereinrichtung ausgebildet, die das aus dem Maximum-Length-Sequence-Signal erzeugte Datensignal in Echtzeit verarbeitet. Hierbei kann die Filtereinheit als Softwaremodul innerhalb der Verarbeitungs- bzw. Steuereinheit oder als eigenständige Hardwarekomponente ausgeführt sein. Die Signalverarbeitung erfolgt vorzugsweise durch digitale Filterstrukturen, insbesondere Butterworth- und Biquad-Filter, die aufgrund ihrer Stabilität und Recheneffizienz für eingebettete Systeme besonders geeignet sind. Die Filterparameter können dabei dynamisch an das jeweilige Messszenario angepasst werden, um den nutzbaren Frequenzbereich, beispielsweise den Ultraschallbereich zwischen 20 kHz und 75 kHz, präzise zu begrenzen. Durch die digitale Implementierung der Filtereinheit wird eine reproduzierbare und verlustarme Signalformung ermöglicht, wodurch sichergestellt ist, dass das Sendesignal über den gewünschten Frequenzbereich eine konstante spektrale Energiedichte aufweist.

In einer bevorzugten Ausgestaltung umfasst die Filtereinheit eine Kombination aus Hochpass-, Tiefpass- und/oder Bandpassfiltern, die in Kaskade oder parallel geschaltet sein können. Hierdurch kann das Maximum-Length-Sequence-Signal gezielt so angepasst werden, dass störende niederfrequente Anteile und unerwünschte harmonische Komponenten unterdrückt werden. Durch den Einsatz von Butterworth-Filtern mit maximal flacher Amplitudencharakteristik im Durchlassbereich wird das MLS-Signal in seiner Phasenlage nur minimal beeinflusst, was für die spätere Kreuzkorrelation und Impulsantwortbestimmung wesentlich ist. Biquad-Filterstrukturen erlauben zusätzlich eine flexible Anpassung der Flankensteilheit und Dämpfung, wodurch das Filterverhalten optimal an die elektroakustischen Eigenschaften der verwendeten Schallwandlereinheit angepasst werden kann. Auf diese Weise trägt die Filtereinheit wesentlich zur Linearität und Genauigkeit des gesamten Messverfahrens bei.

Es ist vorteilhaft, wenn der MEMS-Schallwandler zumindest ein Piezoelement und eine mit dem zumindest einen Piezoelement gekoppelte Membran umfasst, wobei das zumindest eine Piezoelement und die Membran mittels einer Verstärkungsplatte miteinander gekoppelt sind, so dass die Membran zum Erzeugen der Ultraschallwellen flächig ausgelenkt werden kann. Die Anordnung des Piezoelements mit der Membran ermöglicht eine effiziente Erzeugung hochfrequenter Ultraschallwellen, was zur Verbesserung der Signalqualität und zur präzisen Erfassung beiträgt. Mit Hilfe der flächigen Kopplung der Membran mit dem zumindest einen Piezoelement über die Verstärkungsplatte kann ein sehr konstanter Ultraschall mit einem kontinuierlichen Spektrum im Frequenzbereich erzeugt werden.

Gemäß einer vorteilhaften Weiterbildung der Erfindung ist der MEMS-Schallwandler derart angeordnet, dass bei einer bestimmungsgemäßen Verwendung des Kopfhörers die Ultraschallwellen in einen Ohrkanal des Trägers abgestrahlt werden. Die direkte Abstrahlung in den Ohrkanal sorgt für eine erhöhte Signalqualität und ermöglicht die präzise Erfassung der Vitalparameter durch eine spezifische Reflexionsmessung im Ohrkanal.

Vorteilhaft ist es, wenn mit demselben MEMS-Schallwandler die Ultraschallwellen erzeugt und erfasst werden können. Zusätzlich oder alternativ kann der Kopfhörer ein Mikrofon und/oder einen weiteren MEMS-Schallwandler umfassen, mittels dessen die vom MEMS-Schallwandler erzeugten Ultraschallwellen erfasst werden können.

Vorgeschlagen wird außerdem ein Verfahren zum Ermitteln einer Körperfunktion, insbesondere einer Herzfunktion und/oder Atemfunktion, eines Trägers eines Kopfhörers, insbesondere eines On-Ear-Hörers, Over-Ear-Hörers und/oder In-Ear-Hörers. Der Kopfhörer kann hierbei zumindest ein Merkmal der vorangegangenen und/oder nachfolgenden Beschreibung aufweisen. Des Weiteren kann beim Verfahren zumindest ein Merkmal der vorangegangenen und/oder nachfolgenden Beschreibung gemäß dessen Eigenschaften und/oder Bestimmung verwendet werden.

Beim Verfahren werden mittels eines MEMS-Schallwandlers, insbesondere eines Breitband-MEMS-Schallwandlers, des Kopfhörers Ultraschallwellen aus einem Breitbandspektrum erzeugt. Die Ultraschallwellen dienen zur Messung der Körperfunktion. Die Ultraschallwellen stammen hierbei aus einem Breitbandspektrum, wobei der MEMS-Schallwandler diese Ultraschallwellen aus dem Breitbandspektrum erzeugen kann. Dadurch stehen eine Vielzahl an möglichen Frequenzen der Ultraschallwellen zur Messung der Körperfunktion zur Verfügung.

Beim Verfahren werden mittels des MEMS-Schallwandlers, insbesondere des Breitband-MEMS-Schallwandlers, und/oder eines weiteren Mikrofons die Ultraschallwellen erfasst.

Weiterhin wird mittels einer Steuereinheit des Kopfhörers anhand der erzeugten und erfassten Ultraschallwellen die Körperfunktion des Trägers des Kopfhörers ermittelt.

Vorteilhaft ist es, wenn mittels des MEMS-Schallwandlers ein Messsignal mit einer oder mehreren Messfrequenzen aus dem erzeugbaren Breitbandspektrum ausgesendet wird, um eine Körperfunktion zu ermitteln. Diese ermöglicht eine flexible Auswahl der Messfrequenzen innerhalb des Breitbandspektrums, wodurch spezifische Anforderungen an die Analyse und Erfassung der Körperfunktion erfüllt werden können. Zudem können unterschiedliche Frequenzen genutzt werden, um die Signalqualität an verschiedene anatomische und physikalische Bedingungen anzupassen, was die Robustheit und Vielseitigkeit des Systems steigert.

Vorteilhaft ist es, wenn zumindest ein Messsignal erzeugt wird, dessen Ultraschallwellen einen oder mehrere Messfrequenzpeaks in einem Breitbandspektrum aufweisen. Die Erzeugung von Messfrequenzpeaks ermöglicht eine gezielte Analyse, wodurch Störungen minimiert und die Signalqualität verbessert werden können. Dadurch kann das Signal-Rauschen-Verhältnis verbessert werden, da ein schmalbandiges Messsignal ausgesendet wird und Störungen bzw. Hintergrundrauschen leicht erkannt werden können. Dies ist hilfreich für die zuverlässige und effiziente Erkennung bestimmter Muster oder Veränderungen in den reflektierten Signalen. Des Weiteren erzeugt ein ausgesendetes Messsignal mit einem oder mehreren Messfrequenzpeaks im Breitbandspektrum reflektierte Signale, die ebenfalls einen scharfen Peak aufweisen. Die Auswertung wird dadurch vereinfacht.

Zusätzlich oder alternativ ist es von Vorteil, wenn ein Messsignal erzeugt wird, dessen Ultraschallwellen ein, insbesondere kontinuierliches, Messfrequenzspektrum im Breitbandspektrum umfassen. Ein kontinuierliches Messfrequenzspektrum bietet den Vorteil, dass eine breitere Datenbasis geschaffen wird, die eine umfassendere Analyse ermöglicht. Es werden gleichzeitig mehrere Frequenzen gleichzeitig zur Messung ausgesendet, so dass mehrere Messungen gleichzeitig durchgeführt werden können. Durch die Nutzung des gesamten Breitbandspektrums können komplexe Reflexionen und Interaktionen der Ultraschallwellen mit Gewebe oder Oberflächen detaillierter erfasst werden, was die Vielseitigkeit und Anpassungsfähigkeit des Systems für unterschiedliche Anwendungen erhöht. Durch das breite Messfrequenzspektrum im Breitbandspektrum ist außerdem automatisch die Messfrequenz enthalten, die die besten Messergebnisse liefert.

Vorteilhafterweise führt die Steuereinheit bzw. die Verarbeitungseinheit vor der Ermittlung des zumindest einen Merkmals eine Fourier-, Wavelet- oder Periodogramm-Analyse durch. Zusätzlich oder alternativ kann die Verarbeitungseinheit eine Cepstrumanalyse durchführen, um das zumindest eine Merkmal zu ermitteln. Zusätzlich oder alternativ können diese Analysen einzeln oder in Kombination auch für die Authentifikation durchgeführt werden. Diese Analysen dienen der Transformation des Echosignals in den Frequenzbereich, wodurch spektrale Veränderungen identifiziert werden können. So lassen sich Resonanzverschiebungen oder Dämpfungsänderungen erfassen, die physiologische Informationen enthalten.

Vorteilhaft ist es, wenn von der Steuereinheit ein Suchprogramm und/oder Suchalgorithmus ausgeführt wird, mit dem eine zu einem Träger des Kopfhörers passende Messfrequenz ermittelt wird. Durch diese Anpassung an die individuellen Gegebenheiten des Trägers, wie die spezifische Anatomie des Gehörgangs und/oder individuelle Gewebeeigenschaften, wird eine optimale Signalqualität und Messgenauigkeit erreicht. Diese Funktion ermöglicht es, die Messfrequenz des Messsignals dynamisch und/oder automatisch an den Träger anzupassen und/oder zu optimieren, wodurch die Zuverlässigkeit der Erfassung der Körperfunktionen, wie der Herz- oder Atemfrequenz, erhöht werden. Zudem wird der Tragekomfort verbessert, da keine manuelle Kalibrierung oder Anpassung erforderlich ist, was die Bedienung des Systems für den Nutzer vereinfacht und eine konsistente Leistung gewährleistet. Beispielsweise kann das Suchprogramm und/oder der Suchalgorithmus derart erfolgen, dass die Steuereinheit mehrere Messfrequenzen durchprobiert und dann diese auswählt, welche die besten Ergebnisse liefert. Beispielsweise kann die Steuereinheit alle Messfrequenzen von 20 kHz bis 100 kHz in 1 kHz-Schritten durchprobieren.

Darüber hinaus ist es vorteilhaft, wenn Ultraschallwellen erzeugt werden, die im Breitbandspektrum dessen zumindest einer Messfrequenzpeak des Messsignals ein Breite von kleiner als 3 kHz, 2 kHz oder 1 kHz aufweist. Hierdurch sind die Messsignale ausreichend schmalbandig, stammen allerdings aus dem durch den MEMS-Schallwandler erzeugbaren Breitbandspektrum. Durch das scharfe Messsignal kann die Auswertung vereinfacht werden, da auch die reflektierten Ultraschallwellen ein schmalbandiges Spektrum aufweisen.

Zusätzlich oder alternativ können die Ultraschallwellen erzeugt werden, deren Messfrequenzspektrum eine Frequenzbreite von größer als 5 kHz, 10 kHz, 20 kHz, 30 kHz, 40 kHz, 50 kHz, 60 kHz, 70 kHz oder 80 kHz und/oder von kleiner als 80 kHz oder von kleiner als 70 kHz oder von kleiner als 60 kHz oder von kleiner als 50 kHz oder von kleiner als 40 kHz oder von kleiner als 30 kHz oder von kleiner als 20 kHz oder von kleiner als 10 kHz oder von kleiner als 5 kHz aufweist. Hierbei ist es so, dass die obere Grenze größer ist als die untere Grenze. Hierdurch können mehr Informationen bei der Messung gesammelt werden, da mehrere Frequenzen gleichzeitig bzw. das breite Messsignal gemäß dem breiten Messfrequenzspektrum zur Messung ausgesendet werden.

Vorteilhafterweise werden Ultraschallwellen erzeugt, die einen Schalldruckpegel von größer gleich 50 dB, bevorzugt größer gleich 55 dB, besonders bevorzugt größer gleich 70 dB, aufweisen. Ein gleichmäßiger und ausreichend hoher Schalldruckpegel sorgt für eine robuste Signalqualität und ermöglicht eine präzise Analyse auch bei geringem Signal-Rausch-Verhältnis.

Es ist vorteilhaft, wenn Ultraschallwellen erzeugt werden, die einen Schalldruckpegel von kleiner gleich 90 dB, bevorzugt kleiner gleich 85 dB, besonders bevorzugt kleiner gleich 80 dB, aufweisen. Die Begrenzung des Schalldruckpegels minimiert die Belastung für das Gehör und erhöht den Tragekomfort, was die kontinuierliche Überwachung unterstützt. Mit Hilfe eines Schalldruckpegels im Bereich von ungefähr zwischen 50 dB und 90 dB kann die Messung noch zuverlässig durchgeführt werden, ohne dass das Ohr und/oder das Gewebe geschädigt wird.

Vorteilhafterweise weist der Schalldruckpegel, insbesondere über die gesamte Frequenzbreite des Messfrequenzspektrums eine Abweichung von +/- 10 dB, insbesondere von +/- 5 dB, um einen Mittelwert auf. Eine geringe Abweichung im Schalldruckpegel gewährleistet eine stabile und verlässliche Messumgebung für die Vitalparameterüberwachung.

Darüber hinaus ist es vorteilhaft, wenn Ultraschallwellen erzeugt werden, die im Messfrequenzspektrum, insbesondere über seine gesamte Frequenzbreite, ein kontinuierliches Spektrum aufweisen. Ein kontinuierliches Spektrum erlaubt eine hohe Informationsdichte und erhöht die Auswertungsmöglichkeiten, wodurch präzisere und konsistentere Messergebnisse erzielt werden. Durch das kontinuierliche Spektrum stehen im Gegensatz zu einem diskreten Spektrum mehrere Frequenzen zur Messung zur Verfügung.

Es ist vorteilhaft, wenn die Ultraschallwellen mittels eines Signalgenerators erzeugt werden. Die Verwendung eines Signalgenerators ermöglicht die präzise Steuerung und Optimierung der Ultraschallsignale und verbessert so die Messqualität.

Vorteilhaft ist es, wenn zum Erzeugen von hörbaren Schallwellen und Ultraschallwellen die entsprechenden elektrischen Signale mittels eines Modulators zusammengefasst und/oder moduliert werden, wobei dieses modulierte Signal anschließend zur Umsetzung an den MEMS-Schallwandler geleitet wird. Diese Zusammenführung der Signale ermöglicht eine effiziente Nutzung des MEMS-Schallwandlers, der so beide Frequenzbereiche, d.h. die hörbaren Schallwellen und die Ultraschallwellen, gleichzeitig erzeugen kann.

Vorteilhaft ist es, wenn die Messsignale in zeitlichen Abständen zwischen 30 µs und 300 ms, insbesondere zwischen 1 µs und 100 ms, ausgesendet werden. Dieses zeitliche Aussenden der Messsignale ermöglicht eine flexible Anpassung an die spezifischen Anforderungen der Anwendung. Kürzere Abstände zwischen den Signalen erhöhen die zeitliche Auflösung und eignen sich besonders für die präzise Erfassung schneller Veränderungen. Gleichzeitig ermöglichen längere Zeitabstände zwischen den Messsignalen eine Reduzierung des Energieverbrauchs.

Gemäß einer vorteilhaften Weiterbildung der Erfindung werden die Ultraschallwellen im Messspektrum und/oder im Breitbandspektrum mittels eines Chirp-, Sweep- und/oder Multiton-Signals erzeugt. Dadurch können die Ultraschallwellen mit dem besagten Frequenzbereich und/oder mit dem kontinuierlichen Spektrum erzeugt werden.

Des Weiteren ist es vorteilhaft, wenn zum Ermitteln der Körperfunktion mit demselben MEMS-Schallwandler die Ultraschallwellen erzeugt und erfasst werden. Dies reduziert die Anzahl der benötigten Komponenten und erhöht die Effizienz des Systems, was besonders vorteilhaft für kompakte, tragbare Geräte wie Kopfhörer ist. Zusätzlich oder alternativ kann der Kopfhörer einen weiteren MEMS-Schallwandler und/oder ein weiteres Mikrofon umfassen, mittels dem die reflektierten Ultraschallwellen erfasst werden.

Vorteilhaft ist es, wenn mittels, insbesondere demselben, MEMS-Schallwandler die hörbaren Schallwellen und die Ultraschallwellen erzeugt werden, wobei die hörbaren Schallwellen vorzugsweise gleichzeitig und/oder simultan mit den Ultraschallwellen erzeugt werden. Die Möglichkeit, beide Schallarten durch denselben Schallwandler zu erzeugen, reduziert den Bedarf an separaten Komponenten und ermöglicht eine kompakte Bauweise, die insbesondere für tragbare Geräte wie Kopfhörer von Vorteil ist.

Vorteilhaft ist es, wenn mittels des MEMS-Schallwandlers hörbare Schallwellen erzeugt werden, wobei diese vorzugsweise gleichzeitig mit den Ultraschallwellen erzeugt werden. Die gleichzeitige Erzeugung hörbarer und Ultraschallwellen ermöglicht eine vielseitige Nutzung des Schallwandlers und erhöht die Funktionalität des Kopfhörers. Dadurch kann der Kopfhörer weiterhin beispielsweise zum Musikhören verwendet werden, gleichzeitig allerdings ebenfalls zur Messung der Körperfunktionen.

Gemäß einer vorteilhaften Weiterbildung der Erfindung wird die Körperfunktion mittels eines Algorithmus und/oder eines Auswertprogramms ausgewertet, wobei vorzugsweise die Steuereinheit den Algorithmus und/oder das Auswertprogramm ausführt. Die Anwendung von Algorithmen zur Signalverarbeitung erhöht die Genauigkeit und Effizienz bei der Analyse von Herz- und Atemsignalen und verbessert die Zuverlässigkeit des Systems für eine kontinuierliche Überwachung.

Vorgeschlagen wird die Verwendung eines, insbesondere breitbandigen, MEMS-Schallwandlers für einen Kopfhörer und/oder ein Verfahren zum Ermitteln einer Körperfunktion. Der Kopfhörer und/oder das Verfahren weisen hierbei zumindest ein Merkmal der vorangegangenen und/oder nachfolgenden Beschreibung auf. Des Weiteren weist der MEMS-Schallwandler zumindest ein Merkmal der vorangegangenen und/oder nachfolgenden Beschreibung auf, um im Kopfhörer und/oder gemäß dem hier beschriebenen Verfahren verwendet zu werden.

Eine Eigenschaft der Breitband-Ultraschall-Sensorik ist, dass ein System mit Breitband-MEMS-Schallwandlern sehr kurze Pulse erzeugen kann, die sowohl informations- als auch energiedicht sind. Dies ist auf die besonderen Eigenschaften von Breitbandsignalen zurückzuführen.

Die Fourier-Transformation zeigt eine inverse Beziehung bei gepulsten Signalen. Die Dauer eines, insbesondere zeitdiskreten, Pulses ist umgekehrt proportional zu seiner Bandbreite. Ein Breitband-MEMS-Schallwandler kann daher Pulse erzeugen, die sehr kurz sind, was eine hohe zeitliche Messauflösung ermöglicht.

Für die Herz- und/oder Atemüberwachung basiert das Funktionsprinzip auf folgenden Schritten. Der MEMS-Schallwandler sendet kontinuierlich kurze, breitbandige Ultraschallpulse in den Gehörgang. Die Schallwellen interagieren mit dem Luftvolumen und den Geweben im Ohr. Wenn das Blut durch die Gefäße im Ohr pulsiert, führt dies zu kleinen Bewegungen (Volumenänderungen) und Änderungen in der Dichte der Gewebe. Diese Veränderungen beeinflussen die reflektierten Ultraschallwellen.

Das Mikrofon des Kopfhörers, insbesondere der MEMS-Schallwandler, der die Ultraschallwellen aussendet, ein weiteres Mikrofon und/oder ein weiterer MEMS-Schallwandler, erfasst die reflektierten Wellen und nimmt sie mit hoher Abtastrate auf, um die Details der kurzen Pulse zu erfassen. Die Signale werden dann verarbeitet, um diese zu extrahieren. Algorithmen können hierbei Störungen und andere Artefakte herausfiltern, um die Herzfrequenz und eventuelle Anomalien in der Herz- und/oder Atemfunktion, beispielsweise im Herzschlag oder der Atemfrequenz, zu bestimmen.

Breitband-MEMS-Schallwandler bieten für die Messung der Körperfunktionen wesentliche Vorteile. Durch die Erzeugung sehr kurzer Pulse können die Signale mit höherer Auflösung erfasst werden, was zu detaillierteren Informationen über die reflektierten Signale führt. Dies hilft bei der genauen Erkennung der kleinen und schnellen Veränderungen im Gewebe und Blutfluss. Zudem dringen Breitband-Ultraschallwellen in Abhängigkeit von der Frequenz unterschiedlich tief in das Gewebe ein, was zu einer reichhaltigeren Datenerfassung beiträgt, und die Präzision der Herzfrequenzmessung verbessert.

Die Verwendung eines breitbandigen MEMS-Schallwandlers ermöglicht zudem Signalverarbeitungstechniken wie Frequenzbereichsanalyse und adaptive Filterung, was die Fähigkeit zur Extraktion der pulsierenden Komponente aus störanfälligen Daten weiter steigert. Darüber hinaus führt die Nutzung eines Breitbandsignals zu einem verbesserten Signal-Rausch-Verhältnis (SNR), da durch die Frequenzverteilung das eigentliche Pulssignal besser vom Hintergrundrauschen unterschieden werden kann. Dies verbessert die Klarheit und Zuverlässigkeit der Messung der Körperfunktion. Diese Techniken können zusätzliche Informationen aus den empfangenen Signalen extrahieren und verbessern so die Genauigkeit und Zuverlässigkeit der ToF-Messungen. Auch bieten Breitband-Ultraschallwellen die Möglichkeit zur adaptiven Kompensation von Umweltschwankungen, wie etwa Temperatur- und Feuchtigkeitsänderungen, die die Schallgeschwindigkeit beeinflussen und dadurch die Genauigkeit der Laufzeitmessungen beeinträchtigen können.

Dieses Verfahren ist nicht-invasiv und bietet Komfort bei der kontinuierlichen Überwachung, da niedrige Schalldruckpegel genutzt werden können, was potenzielles Unbehagen für den Träger vermeidet. Durch die hohe Energiedichte der Pulse können die Schalldruckpegel begrenzt werden, ohne an Messqualität einzubüßen, wodurch die Nutzerfreundlichkeit gesteigert wird.

Die Funktionsweise ist außerdem resistenter gegenüber leichten Positionsänderungen des Sensors, so dass auch bei nicht perfekter Platzierung des Geräts konsistente und genaue Messungen gewährleistet werden. Die durch Breitband-Ultraschall erzeugte hohe Informationsdichte verbessert zudem den Kalibrierungsprozess, da genauere Anpassungen an individuelle Unterschiede im Ohrkanal und den Gewebeeigenschaften möglich sind.

Der Breitband-Ultraschall-MEMS-Schallwandler verbessert hierbei die Handhabung von Mehrwegeeffekten (wo Signale von mehreren Oberflächen reflektiert werden), indem direkte Signalwege von reflektierten Signalpfaden anhand ihres Frequenzinhalts unterschieden werden können. Dies verbessert die Genauigkeit der Erkennung relativer Positionen im Raum.

Die kurze Dauer der Pulse ermöglicht es dem System, schärfere und detailliertere Bilder zu erzeugen. Kurze Pulse bedeuten auch eine geringere Pulsbreite in Bezug auf die Entfernung, was zur Auflösung kleinerer Merkmale und Bewegungen beiträgt.

Breitband-Ultraschall-Signale sind außerdem weniger anfällig für Interferenzen und Störungen im Vergleich zu Schmalbandsignalen, was die Klarheit des empfangenen Signals erhöht - ein entscheidender Vorteil für die präzise Messung der Laufzeit.

Breitband-MEMS-Schallwandler bieten eine höhere Auflösung und Genauigkeit für Time-of-Flight-Anwendungen. Eine verbesserte Handhabung von Mehrwege- und Reflexionseffekten, höhere Signalqualität, präzisere zeitliche und räumliche Auflösung, bessere Materialinteraktion und fortschrittliche Signalverarbeitungsmöglichkeiten verbessern zusammen die Fähigkeit des Systems, Gesten genau zu erkennen, Entfernungen zu messen und Objekte in verschiedenen Umgebungen zu charakterisieren.

Ein breitbandiger MEMS-Schallwandler kann Ultraschallwellen in einem weiten Frequenzbereich erzeugen. Das bedeutet, dass der Schallwandler nicht nur eine einzige, festgelegte Frequenz ausstrahlt, sondern Schallwellen über ein, insbesondere kontinuierliches, Spektrum hinweg abgibt - zum Beispiel von zumindest 20 kHz bis 40 kHz oder bis 60 kHz oder bis 80 kHz oder bis 100 kHz. Dieses Spektrum kann hierbei ein kontinuierliches Spektrum sein, insbesondere im Messspektrum. Diese Vielzahl an Frequenzen bzw. das kontinuierliche Frequenzspektrum verbessert die Auflösung der Signale, da verschiedene Frequenzen unterschiedlich tief ins Gewebe eindringen oder von verschiedenen Oberflächen unterschiedlich reflektiert werden. So können feinere Details erfasst und analysiert werden, was insbesondere für die Überwachung der Herz- oder Atemfunktionen hilfreich ist.

Ein kurzer Puls im Ultraschallbereich erlaubt es, schnelle Änderungen im Körper - wie den Herzschlag - exakt zu erkennen und zu analysieren. Durch die Vielzahl der Frequenzen werden die Reflexionssignale reicher an Informationen, was die Genauigkeit beispielsweise der Herzfrequenz- oder Atemfrequenzmessung erhöht. Außerdem wird durch die Bandbreite des Signals das Hintergrundrauschen (also Störgeräusche) reduziert, wodurch das eigentliche Signal leichter und zuverlässiger zu erfassen ist.

Ein breitbandiger MEMS-Schallwandler ist zudem widerstandsfähiger gegenüber leichten Positionsänderungen des Sensors. Selbst wenn der Kopfhörer oder das Headset im Ohr etwas verrutscht, kann der Schallwandler weiterhin präzise Messungen durchführen. Das ist gerade bei tragbaren Geräten ein großer Vorteil, da das Gerät nicht exakt positioniert sein muss, um genaue Ergebnisse zu liefern. Außerdem kann die zusätzliche Datenvielfalt helfen, das Gerät auf individuelle Unterschiede im Ohrkanal des Nutzers anzupassen und so eine bessere Kalibrierung und Genauigkeit zu gewährleisten.

Vorgeschlagen wird und vorteilhaft ist ferner die Verwendung eines Maximum-Length-Sequence-Signals zum Ermitteln zumindest eines Merkmals eines Trägers und/oder zur Authentifikation, wobei das Maximum-Length-Sequence-Signal vorzugsweise gemäß der vorangegangenen und/oder nachfolgenden Beschreibung ausgebildet und/oder erzeug wird. Das Maximum-Length-Sequence-Signal wird für das Verfahren gemäß der vorangegangenen und/oder nachfolgenden Beschreibung verwendet. Der technische Vorteil der Verwendung besteht darin, dass das MLS-Signal unabhängig von der Anatomie und der Anordnung des Geräts im Gehörgang einsetzbar ist, wodurch Kalibrierverfahren entfallen. Mit Hilfe des MLS-Signals können die ausgesendeten Schallwellen erzeugt werden, mittels denen die Messung bzw. Bestimmung durchgeführt wird.

Das Maximum-Length-Sequence-Signal (MLS) ist ein deterministisches, pseudozufälliges digitales Signal, das durch eine Bitfolge definiert ist, die mittels eines linearen Feedback-Shift-Registers (LFSR) erzeugt wird. Dieses Schieberegister generiert eine Sequenz mit maximaler Periodenlänge von
2^*n*-1, wobei n die Bitzahl des Registers ist. Die Bitzahl liegt vorzugsweise im Bereich von 8 bis 16, wodurch ein ausgewogenes Verhältnis zwischen Signalauflösung, Periodendauer und Rechenaufwand erzielt wird.

Das MLS-Signal besteht aus einer zeitabhängigen Folge diskreter Werte, die zwei Zustände annehmen, typischerweise 0 und 1. Für die digitale Weiterverarbeitung wird das Signal in eine symmetrische Form überführt, bei der die Werte um den Nullpunkt zentriert sind, beispielsweise -1 und +1. Dies erlaubt die mathematisch stabile Anwendung digitaler Filter und die lineare Signalverarbeitung in Fließkommadarstellung. Jeder Wert des Signals wird in einem festen zeitlichen Abstand ausgegeben, der durch die Abtastrate bestimmt ist. Typische Abtastraten liegen zwischen 50 kHz und 200 kHz, vorzugsweise bei 96 kHz, wodurch ein nutzbarer Frequenzbereich bis zu 48 kHz erreicht wird.

Das Maximum-Length-Sequence-Signal zeichnet sich durch ein flaches Frequenzspektrum aus. Es weist eine nahezu gleichmäßige spektrale Energiedichte über den gesamten genutzten Frequenzbereich auf. Dadurch werden alle Frequenzkomponenten gleichzeitig und mit gleicher Energie angeregt, was eine vollständige und gleichzeitige Erfassung der Übertragungsfunktion eines Systems ermöglicht. Die Autokorrelationsfunktion des MLS-Signals ergibt näherungsweise einen Dirac-Impuls beziehungsweise eine Kronecker-Delta-Funktion. Durch Kreuzkorrelation des ausgesendeten und des empfangenen Signals kann somit die Impulsantwort des Systems präzise rekonstruiert werden. Diese Eigenschaft ist für breitbandige Messverfahren von besonderem Vorteil, da die vollständige Systemantwort in einem einzigen Messzyklus ermittelt werden kann.

Zur Anpassung an das jeweilige Messszenario wird das digitale MLS-Signal gefiltert. Die Filterung erfolgt vorzugsweise durch eine digitale Filtereinheit, die das Signal frequenzselektiv anpasst und für den Messbereich optimiert. Die Filtereinheit kann eine Kombination aus Hochpass-, Tiefpass- und Bandpassfiltern umfassen, die in Kaskade oder parallel geschaltet sind. Vorzugsweise kommen digitale Butterworth-Filter mit flacher Amplitudencharakteristik oder Biquad-Filterstrukturen mit einstellbarer Flankensteilheit und Dämpfung zum Einsatz. Diese Filterstrukturen gewährleisten eine verlustarme und phasenstabile Signalverarbeitung.

Die Filterung dient insbesondere dazu, das Frequenzspektrum des Signals auf den gewünschten Arbeitsbereich zu begrenzen. Hierbei werden hörbare Frequenzen entfernt, sodass das Signal in den Ultraschallbereich verschoben wird. Der untere Grenzfrequenzbereich kann zwischen 20 kHz und 75 kHz liegen, während die obere Grenzfrequenz vorzugsweise zwischen 40 kHz und 100 kHz eingestellt wird. Dadurch wird das akustische Sendesignal für den Benutzer unhörbar, während gleichzeitig eine hohe spektrale Auflösung und Messpräzision erzielt wird. Die resultierende Signalform ist ein geglättetes, kontinuierliches digitales Sendesignal mit gleichmäßiger Energieverteilung über den gewünschten Frequenzbereich.

Neben der Bandbegrenzung kann die Filtereinheit eine frequenzabhängige Vorverzerrung des Signals durchführen, um den nichtlinearen Frequenzgang und die frequenzabhängige Empfindlichkeit der elektroakustischen Wandler, insbesondere von Lautsprecher und Mikrofon, zu kompensieren. Hierdurch wird eine Linearisierung der gesamten Übertragungsfunktion des Systems erreicht, was die Genauigkeit der Impulsantwortbestimmung erhöht.

Das durch die Filtereinheit erzeugte digitale Sendesignal weist eine geglättete, nahezu sinusähnliche Struktur auf und kann über einen Digital-Analog-Wandler in ein analoges elektrisches Sendesignal gewandelt werden. Dieses wird anschließend von einer Schallwandlereinheit, beispielsweise einer MEMS-Schallwandlereinheit, als akustisches Signal abgestrahlt. Das gefilterte MLS-Signal bleibt trotz der Glättung deterministisch und periodisch, sodass es für die Kreuzkorrelationsanalyse weiterhin eindeutig identifizierbar ist.

Ein wesentlicher technischer Vorteil des Maximum-Length-Sequence-Signals besteht darin, dass es ein breitbandiges, kalibrierfreies und robustes Anregungssignal darstellt. Da alle Frequenzen gleichzeitig und gleichmäßig angeregt werden, ist keine individuelle Kalibrierung auf den Benutzer oder die Geometrie des Gehörgangs erforderlich. Anatomische Unterschiede oder Positionsänderungen des Geräts wirken sich nur auf die Form der gemessenen Impulsantwort aus, ohne das Verfahren zu beeinträchtigen.

Darüber hinaus verteilt das MLS-Signal unvermeidbare Intermodulationsverzerrungen über ein breites Spektrum. Diese Verzerrungsprodukte liegen damit unterhalb der Hörschwelle und sind messtechnisch unkritisch. Dadurch wird eine hohe Störfestigkeit erreicht, und das Signal bleibt auch bei nichtlinearem Systemverhalten präzise auswertbar.

Die mathematische Struktur des Signals ermöglicht zudem eine effiziente Implementierung. Das MLS kann mit geringem Rechenaufwand in Software oder Hardware erzeugt werden und eignet sich dadurch für eingebettete Systeme mit begrenzten Ressourcen. Die Wiederholung des Signals nach jeder Periodenlänge ermöglicht eine kontinuierliche, zeitlich fortlaufende Messung, bei der Änderungen der Impulsantwort oder des Frequenzgangs in Echtzeit erkannt werden können.

Das Maximum-Length-Sequence-Signal ist somit ein geeignetes Anregungssignal für breitbandige Messverfahren, insbesondere zur Bestimmung von Übertragungsfunktionen, Impulsantworten und physiologischen oder biometrischen Merkmalen. Es kombiniert hohe spektrale Abdeckung, Reproduzierbarkeit, Robustheit und Kalibrierfreiheit in einem einzigen deterministischen Signalprinzip.

Weitere Vorteile der Erfindung sind in den nachfolgenden Ausführungsbeispielen beschrieben. Es zeigen:
- **Figur 1**: eine schematische Schnittansicht eines Kopfhörers in Form eines In-Ear-Hörers mit einem MEMS-Schallwandler,
- **Figur 2**: eine schematische Schnittansicht eines Kopfhörers in Form eines Over-Ear-Hörers bzw. On-Ear-Hörers mit einem MEMS-Schallwandler,
- **Figur 3**: ein beispielhaftes Breitbandspektrum im Ultraschallbereich eines Breitband-MEMS-Schallwandlers,
- **Figur 4**: ein beispielhaftes vom Breitband-MEMS-Schallwandler ausgesandtes Messsignal zur Messung der Körperfunktion, bei dem das Messsignal eine Frequenzpeak ist,
- **Figur 5**: ein beispielhaftes vom Breitband-MEMS-Schallwandler ausgesandtes Messsignal zur Messung der Körperfunktion, bei dem das Messsignal ein breites Frequenzspektrum ist,
- **Figur 6**: eine schematische Schnittansicht eines MEMS-Schallwandlers mit einem Piezoelement, das mit einer Membran gekoppelt ist,
- **Figur 7**: eine schematische Schnittansicht eines MEMS-Schallwandlers mit zwei Piezoelementen, die beide mit der Membran gekoppelt sind.

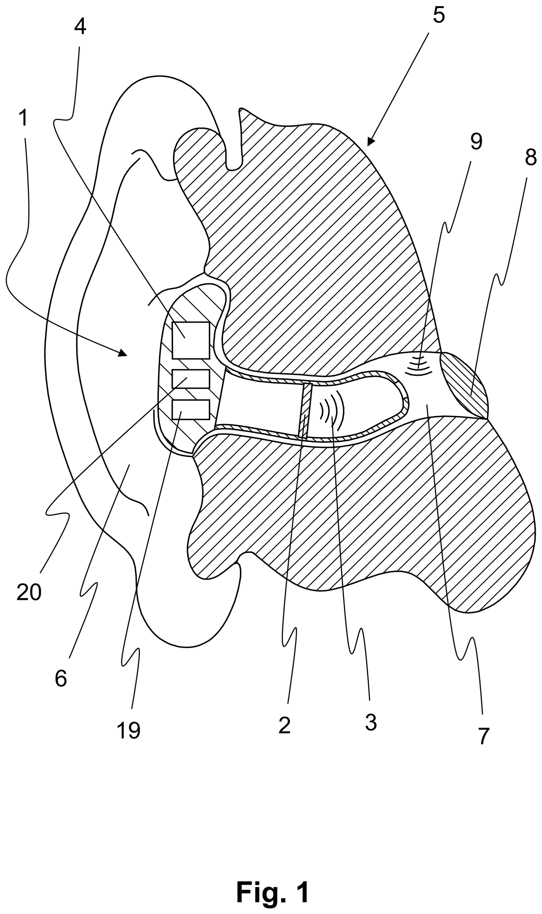

Das in Figur 1 dargestellte Ausführungsbeispiel zeigt einen Kopfhörer 1 in Form eines In-Ear-Hörers, der in einem Ohr 6 eines Trägers 5 angeordnet ist. Der Kopfhörer 1 ist hier zumindest teilweise in einen Gehörgang 7 eingeführt. Der Kopfhörer 1 umfasst einen Schallwandler 2, der ein MEMS-Schallwandler 2 ist und zur Erzeugung und Erfassung von Ultraschallwellen 3 dient. Der Schallwandler 2 ist dabei derart angeordnet, dass die erzeugten Ultraschallwellen 3 in den Gehörgang 7 des Ohrs 6 abgestrahlt werden.

Wie aus dem Ausführungsbeispiel der Figur 1 hervorgeht, umfasst der Kopfhörer 1 eine Steuereinheit 4, die die erfassten Ultraschallwellen 3 verarbeitet, um Körperfunktionen des Trägers 5 zu ermitteln. Diese Körperfunktionen sind beispielsweise die Herz- und Atemfunktionen, deren Erfassung durch die Analyse der von Gewebestrukturen reflektierten Ultraschallwellen 9 ermöglicht wird.

Der Kopfhörer 1 umfasst weiterhin einen Signalgenerator 19, der verschiedene Signalformen wie Chirp-, Sweep- oder Multiton-Signale erzeugen kann, die zur Ausbildung der Ultraschallwellen 3 verwendet werden. Mit Hilfe dieser Signalformen können Ultraschallwellen 3 mit beispielsweise einem kontinuierlichen Spektrum erzeugt werden.

Gemäß Figur 1 ist der Schallwandler 2 derart ausgelegt, dass er Schallwellen 3 aus einem Breitbandspektrum 10 erzeugen kann. Das Breitbandspektrum 10 kann sich beispielsweise über einen Frequenzbereich 3 kHz bis zu 80 kHz erstrecken. Diese breite Frequenzabdeckung bietet den Vorteil, dass unterschiedliche Frequenzen zur Messung verwendet werden können. Die Verwendung des Breitbandspektrums 10 kann das Signal-Rausch-Verhältnis verbessern.

Zusätzlich oder alternativ kann der Kopfhörer 1 Ultraschallwellen 3 mit einem Schalldruckpegel 12 im Bereich des Breitbandspektrums 10 ausbilden, der bevorzugt zwischen 50 dB und 80 dB liegt. Diese Begrenzung des Schalldruckpegels 12 trägt zur Stabilität der Messungen bei und sorgt für eine komfortable Anwendung, indem sie die Belastung des Gehörs minimiert.

Des Weiteren umfasst der hier gezeigte Kopfhörer 1 einen Modulator 20. Mit Hilfe des Modulators 20 können gleichzeitig Schallwellen 3 im hörbaren Frequenzspektrum, also hörbare Schallwellen 3, und im Ultraschallspektrum, also Ultraschallwellen 3, erzeugt werden. Der Modulator 20 kann hierbei die entsprechenden elektrischen Signale zusammenfassen bzw. miteinander modulieren, so dass der MEMS-Schallwandler 2 daraus die hörbaren Schallwellen und die Ultraschallwellen 3 erzeugt. Es kann somit gleichzeitig Musik gehört und die Messung der Körperfunktion durchgeführt werden.

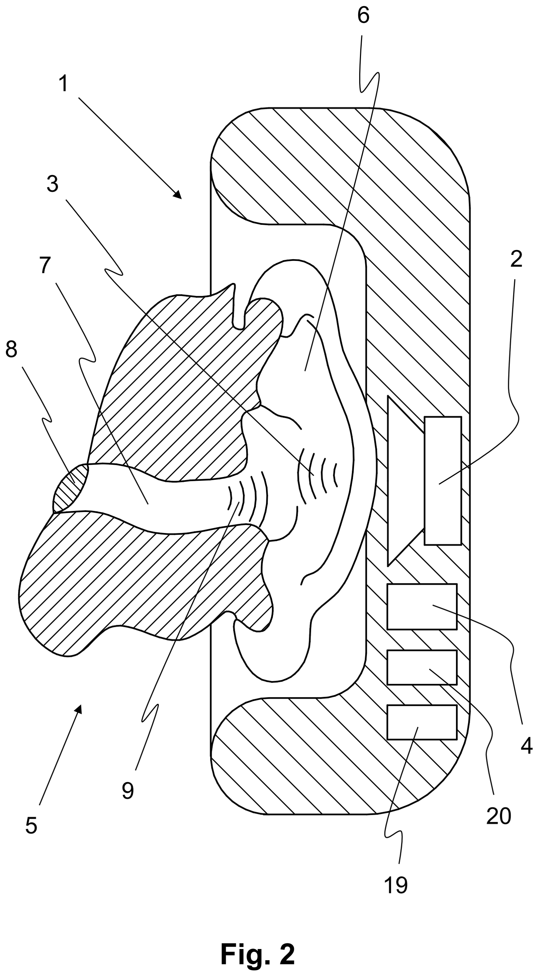

Das in Figur 2 dargestellte Ausführungsbeispiel zeigt einen Kopfhörer 1, der als Over-Ear-Hörer oder On-Ear-Hörer ausgeführt ist. Der Kopfhörer 1 umfasst einen MEMS-Schallwandler 2, der zur Erzeugung und Erfassung von Ultraschallwellen 3 vorgesehen ist. Diese Ultraschallwellen 3 werden in den Gehörgang 7 des Ohrs 6 eines Trägers 5 abgestrahlt, wodurch es möglich ist, die Reflexionen dieser Wellen an Gewebestrukturen zu detektieren. Die reflektierten Ultraschallwellen 9 werden durch den Schallwandler 2 erfasst und zur weiteren Analyse bereitgestellt.

Wie in Figur 2 ersichtlich, ist eine Steuereinheit 4 in den Kopfhörer 1 integriert. Diese Steuereinheit 4 dient zur Auswertung der vom Trommelfell 8 und/oder den umgebenden Gewebestrukturen reflektierten Ultraschallwellen 9. Dadurch wird es möglich, Körperfunktionen des Trägers 5, insbesondere die Herz- und Atemfunktionen, zu überwachen. Die Verwendung von Ultraschallwellen 3 zur Ermittlung dieser Vitalparameter ermöglicht eine präzise Erfassung kleiner Gewebe- und Blutflussveränderungen, was eine detaillierte Analyse der Herz- und Atemsignale unterstützt.

Zusätzlich umfasst der Kopfhörer 1 einen Signalgenerator 19, der verschiedene Signaltypen, wie Chirp- und Sweep-Signale, erzeugen kann. Mit Hilfe dieser Signale können Messsignale 21 mittels der Ultraschallwellen 3 erzeugt werden. Zusätzlich oder alternativ kann das Messspektrum einen Schalldruckpegel 12 mit einer Abweichung von +/- 10 dB, insbesondere +/- 5 dB, aufweisen. Dies verbessert die Erkennung von Veränderungen im Blutfluss und der Gewebebewegungen, was insbesondere für die zuverlässige Überwachung der Herz- und Atemfunktion von Vorteil ist.

Der MEMS-Schallwandler 2 ist ferner derart ausgestaltet, dass er Ultraschallwellen 3 im Breitbandspektrum 10 erzeugen kann. Ein solches Breitbandspektrum 10 erhöht die Flexibilität in der Signalverarbeitung und ermöglicht eine differenzierte Erfassung verschiedener Gewebestrukturen mittels verschiedener Messfrequenzen. Dies verbessert die Detektion kleinerer Bewegungen und sorgt für eine höhere Messgenauigkeit.

Zusätzlich oder alternativ ist der Kopfhörer 1 so ausgelegt, dass die erzeugten Ultraschallwellen einen Schalldruckpegel 12 aufweisen, der vorzugsweise zwischen 50 dB und 80 dB liegt. Diese Begrenzung des Schalldruckpegels 12 trägt zur Stabilität der Messungen bei und minimiert die Belastung des Gehörs, was den Tragekomfort für den Träger 5 erhöht und eine längere Nutzung des Kopfhörers 1 ermöglicht.

Das Ausführungsbeispiel der Figur 2 zeigt somit einen Kopfhörer 1, der mit Ultraschalltechnologie und einer Steuereinheit 4 ausgestattet ist, um die Vitalfunktionen des Trägers 5 präzise zu erfassen. Die Kombination aus Breitband-Ultraschall und einem optimierten Schalldruckpegel 12 bietet eine zuverlässige Lösung für die kontinuierliche Überwachung der Körperfunktionen des Trägers 5.

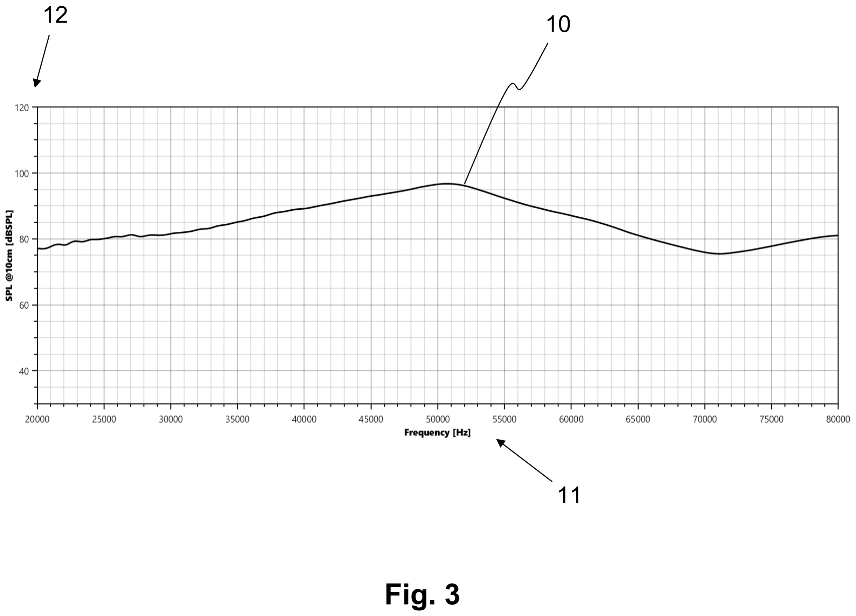

Die Figur 3 zeigt ein beispielhaftes durch den MEMS-Schallwandler 2 erzeugbares Breitbandspektrum 10. Auf der horizontalen Achse ist die Frequenz 11 in Hertz (Hz) abgebildet, während die vertikale Achse den Schalldruckpegel 12 in Dezibel (dBSPL = "sound pressure level" in dB) anzeigt. Das hier gezeigte Breitbandspektrum 10 reicht in diesem Beispiel von etwa 20 kHz bis 80 kHz. Mit Hilfe des MEMS-Schallwandlers 2 können insbesondere auch geringere Frequenzen 11 erzeugt werden. So kann das Breitbandspektrum 10 auch bis hinunter zu 3 kHz reichen, so dass nicht nur Ultraschallwellen 3, sondern auch hörbare Schallwellen 3 erzeugt werden können.

Die Abdeckung eines breiten Frequenzbereichs, insbesondere im Bereich von 3 kHz bis 80 kHz, bietet Vorteile bei der Erfassung unterschiedlicher Gewebearten und ermöglicht eine höhere Auflösung der Messdaten.

Ein solches Breitbandspektrum 10 verbessert die Fähigkeit, die Messung der Körperfunktion an verschiedene Träger 5 des Kopfhörers 1 anzupassen. Verschiedene Träger 5 des Kopfhörers 1 weisen auch andere Formen der Gehörgänge 7 auf. Auf die verschiedenen Formen der Gehörgänge 7 kann reagiert werden, indem aus dem Breitbandspektrum 10 eine andere Messfrequenz gewählt wird, die für die spezielle Form des Gehörgangs 7 besonders gut geeignet ist. Des Weiteren kann es sein, dass der Kopfhörer 1 nicht, wie in den Figuren 1 und 2 gezeigt, angeordnet ist. Gerade beim Sport kann es vorkommen, dass sich der Kopfhörer 1 verschiebt. Dann kann es passieren, dass die Messfrequenz, mit der die Ultraschallwellen 3 zum Messen ausgesendet werden, kein optimales Messergebnis liefert. Durch den MEMS-Schallwandler 2 besteht die Möglichkeit, eine Vielzahl an Messfrequenzen aus dem Breitbandspektrum 10 zu verwenden, um die Messung durchzuführen.

Des Weiteren ist hier ein Schalldruckpegel 12 gezeigt, der die vom MEMS-Schallwandler 2 erzeugbaren Schallwellen 3 aufweisen kann. Gemäß dieser Figur 3 weisen die Schallwellen 3 einen Schalldruckpegel 12 von mindestens 50 dB auf, vorzugsweise auch größer gleich 70 dB und kleiner gleich 100 dB oder kleiner gleich 90 dB, so dass eine ausreichende Signalqualität gewährleistet ist.

Das in Figur 3 dargestellte Breitbandspektrum 10 eines MEMS-Schallwandlers 2 zeigt die Bandbreite der Frequenz 11 und den Schalldruckpegel 12 im Ultraschallbereich, der von etwa 20 kHz bis 80 kHz reicht. Der MEMS-Schallwandler 2 kann in diesem breiten Frequenzbereich bzw. Breitbandspektrum 10 die Schallwellen 3 erzeugen, wodurch eine präzise Erfassung von Gewebe- und Blutreflexionen möglich wird, die für die Überwachung von Vitalparametern, wie Herz- und Atemfunktion, relevant sind. Besonders vorteilhaft ist der flexible Einsatz des Breitbandspektrums 10, da die verwendeten Messfrequenzen auf spezifische Messanforderungen abgestimmt werden können.

Die breite Abdeckung des Ultraschallbereichs ermöglicht eine flexible Frequenzanpassung und/oder Wahl der Messfrequenz für die Messungen und kann helfen, verschiedene Gewebestrukturen zu erkennen, was insbesondere im Gehörgang 7 des Trägers 5 von Vorteil ist. Zusätzlich ermöglicht die Wahl der Messfrequenz aus dem Breitbandspektrum 10 eine Verbesserung des Signal-Rausch-Verhältnisses. Es kann die für die Messung optimale Messfrequenz aus dem Breitbandspektrum 10 gewählt werden, wobei die Wahl der Messfrequenz von der Anatomie des Trägers 5 und der Anordnung des Kopfhörers 1 abhängt.

Mit Hilfe des MEMS-Schallwandlers 2 kann das hier gezeigte kontinuierliche Breitbandspektrum 10 erzeugt werden.

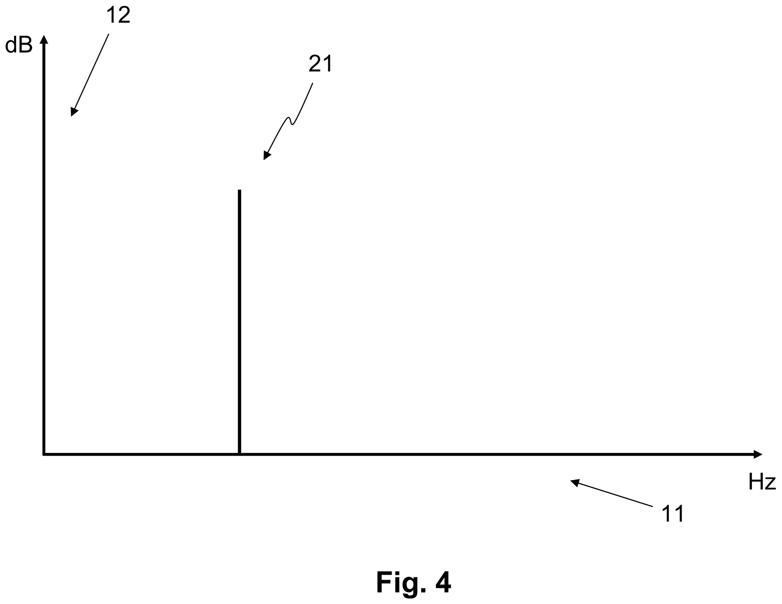

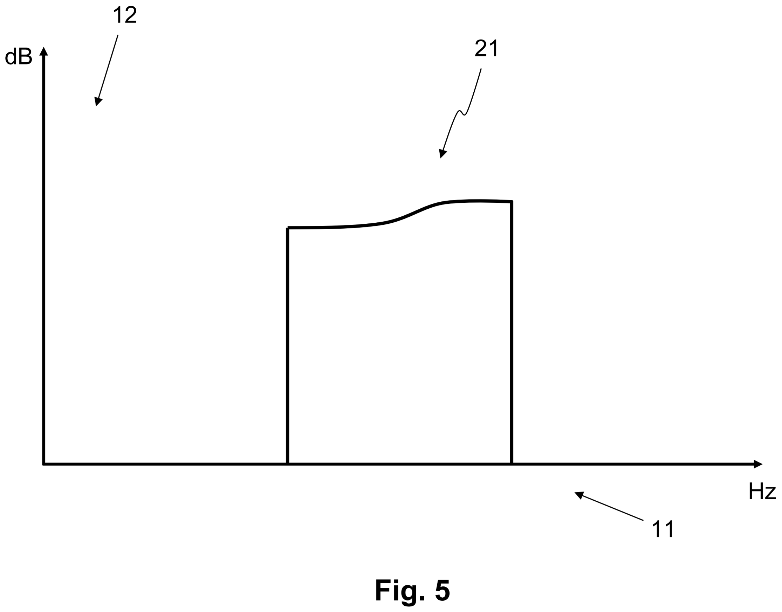

Die beiden Figuren 4 und 5 zeigen jeweils Messsignale 21, mittels derer die Körperfunktion ermittelt werden kann.

Die Figur 4 zeigt ein Messsignal 21 mit einer Messfrequenz, die einen Messfrequenzpeak aufweist. Das heißt, als Messsignal 21 werden Ultraschallwellen 3 ausgesendet, die eine spezifische Frequenz 11 aufweisen. Das Messsignal 21 der beiden Figuren 4 und 5 ist hierbei schematisch dargestellt. Natürlich weist auch dieses Messsignal 21 eine gewisse Breite auf, die beispielsweise 3 kHz betragen kann. Auch werden bei der Erzeugung dieses Messsignals 21 noch andere Ultraschallwellen 3 mit anderen Frequenzen 11 erzeugt, die allerdings einen geringeren Schalldruckpegel 12 aufweisen. Dieses in Figur 4 gezeigte Messsignal 21 kann beispielsweise eine Messfrequenz von 30 kHz aufweisen. Diese 30 kHz können auf eine bestimmte Anatomie des Trägers 5 und/oder auf eine bestimmte Trageanordnung des Kopfhörers 1 abgestimmt sein. Bei einem anderen Träger 5 des Kopfhörers 1 kann das Messsignal 21 beispielsweise 38,5 kHz aufweisen, da die Anatomie unterschiedlich ist. Das vom MEMS-Schallwandler 2 ausgesendete Messsignal 21 kann auch mehrere Messfrequenzen mit entsprechenden Messfrequenzpeaks aufweisen. Dadurch können gleichzeitig mehrere Messungen durchgeführt werden.

Die Figur 5 zeigt das Messsignal 21, welches ein Messfrequenzspektrum aufweist. Mit Hilfe des MEMS-Schallwandlers 2 wird zur Messung der Körperfunktion das in Figur 5 gezeigte Messsignal 21 ausgesendet, welches ein breites Messfrequenzspektrum aufweist. Beispielsweise erstreckt sich das Messsignal 21 von 20 kHz bis 45 kHz. Mittels dieses breiten Messfrequenzspektrums können gleichzeitig mehrere Informationen und/oder Messungen durchgeführt werden.

Wie in der Figur 5 zu sehen ist, weist das Messsignal 21 ein kontinuierliches Messfrequenzspektrum auf, so dass die vielfältigen Informationen und/oder Messungen erzielt werden können. Des Weiteren kann das Messsignal 21 mit dem hier gezeigten Messfrequenzspektrum einen Schalldruckpegel 12 aufweisen, der eine Abweichung von +/-10 dB, insbesondere +/- 5 dB, aufweist. Auch das hier in Figur 5 gezeigte Messsignal 21 kann in der Frequenz 11 an den Träger 5 angepasst werden. In der Figur 5 ist lediglich ein Messsignal 21 gezeigt. Alternativ kann die Messung auch mit mehreren Messsignalen 21 mit jeweils einem Messfrequenzspektrum durchgeführt werden.

Für die Einstellung und/oder Wahl der optimalen Frequenz 11 des Messsignals 21 kann die Steuereinheit 4 beispielsweise ein Suchprogramm und/oder einen Suchalgorithmus durchführen. Dieser kann beispielsweise verschiedene Messfrequenzen des Messsignals 21 durchprobieren und auswerten. Die Steuereinheit 4 kann dann die optimale Messfrequenz des Messsignals 21 auswählen, bei welcher die besten Ergebnisse erzielt werden. Beispielsweise kann die Steuereinheit 4 die Messfrequenz wählen, die das größte Signal-Rausch-Verhältnis aufweist.

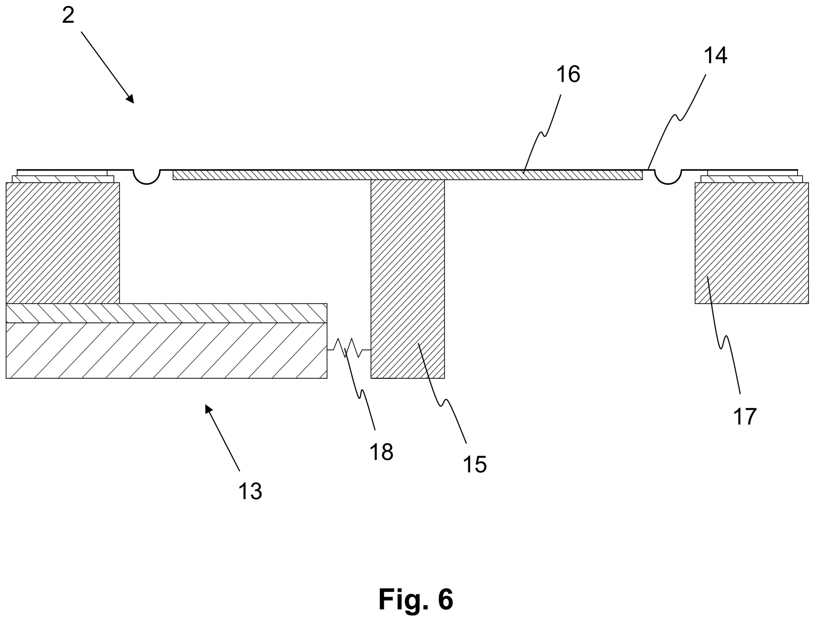

Das in Figur 6 dargestellte Ausführungsbeispiel zeigt eine schematische Schnittansicht eines MEMS-Schallwandlers 2, der ein Piezoelement 13 umfasst, das mit einer Membran 14 gekoppelt ist. Das Piezoelement 13 dient zur Erzeugung von Schwingungen, die durch die Membran 14 als Ultraschallwellen 3 abgegeben werden. Diese Anordnung ermöglicht eine effiziente Erzeugung hochfrequenter Schallwellen 3, die für die präzise Erfassung von Körperfunktionen, wie Herz- und Atemsignalen, genutzt werden können.

Wie aus Figur 6 hervorgeht, ist die Membran 14 über eine Verstärkungsplatte 16 mit dem Piezoelement 13 verbunden. Die Verstärkungsplatte 16 sorgt dafür, dass die Schwingungen des Piezoelements 13 gleichmäßig auf die Membran 14 übertragen werden. Dadurch kann die Membran 14 flächig ausgelenkt werden, was eine gleichmäßige und stabile Abstrahlung der Ultraschallwellen 3 ermöglicht. Die Verstärkungsplatte 16 verbessert somit die Schallabstrahlungsqualität und trägt zur Steigerung der Effizienz und Reichweite des MEMS-Schallwandlers 2 bei. Die flächige Auslenkung kann ferner die breitbandigen Ultraschallwellen 3 erzeugen.

Des Weiteren ist das Piezoelement 13 mittels eines Federelements 18 mit dem Koppelelement 15 verbunden.

Des Weiteren ist der Schallwandler 2 auf einer Trägereinheit 17 angeordnet, die eine stabile Basis für das gesamte System bildet und die Bauteile, wie das Piezoelement 13, die Membran 14, die Verstärkungsplatte 16 und das Federelement 18, in einer festen Position hält.

Die in Figur 6 gezeigte Ausführung des MEMS-Schallwandlers 2 zeigt somit eine kompakte und effiziente Bauweise, bei der das Zusammenspiel zwischen Piezoelement 13, Membran 14, Verstärkungsplatte 16, Federelement 18 und Trägereinheit 17 eine hohe Schallleistung und präzise Signalübertragung ermöglicht. Die Kombination dieser Komponenten gewährleistet eine verbesserte Signalqualität und die Fähigkeit zur Erzeugung eines gleichmäßigen, kontinuierlichen und/oder stabilen Ultraschallspektrums, das für die kontinuierliche Überwachung von Vitalparametern des Trägers 5 des Kopfhörers 1 vorteilhaft ist.

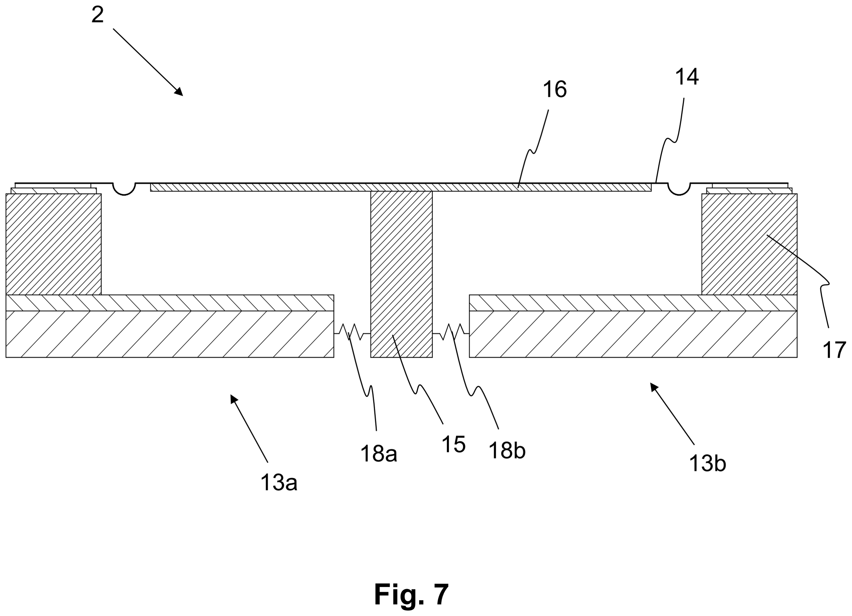

Das in Figur 7 dargestellte Ausführungsbeispiel zeigt eine schematische Schnittansicht eines MEMS-Schallwandlers 2, der zumindest zwei Piezoelemente 13a und 13b umfasst, die jeweils mit der gemeinsamen Membran 14 gekoppelt sind. Die Anordnung der beiden Piezoelemente 13a und 13b ermöglicht eine symmetrische Anregung der Membran 14, was zu einer gleichmäßigen und stabilen Abstrahlung der erzeugten Ultraschallwellen 3 führt.

Die Membran 14 ist über eine Verstärkungsplatte 16 mit den Piezoelementen 13a und 13b verbunden. Die Verstärkungsplatte 16 sorgt dafür, dass die Schwingungen der Piezoelemente gleichmäßig auf die Membran 14 übertragen und verstärkt werden. Zwischen den Piezoelementen 13a und 13b und dem Koppelelement 15 ist hier jeweils ein Federelement 18. Jedes Piezoelement 13a, 13b ist mittels eines Federelements 18 mit dem Koppelelement 15 verbunden.

Mit Hilfe der in den Figuren 6 und 7 gezeigten MEMS-Schallwandler 2 können die Ultraschallwellen 3 erzeugt und/oder erfasst werden. Beispielsweise können mit demselben MEMS-Schallwandler 2 die Ultraschallwellen 3 erzeugt und die reflektierten Ultraschallwellen 9 erfasst werden. Der Kopfhörer 1 kann aber auch zumindest zwei MEMS-Schallwandler 2 umfassen, so dass mit einem ersten MEMS-Schallwandler 2 die Ultraschallwellen 3 erzeugt und mit einem zweiten MEMS-Schallwandler 2 die reflektierten Ultraschallwellen 9 erfasst werden. Mit Hilfe des zumindest einen MEMS-Schallwandlers 2 können auch die hörbaren Schallwellen erzeugt werden, so dass beispielsweise Musik abgespielt werden kann. Da die Ultraschallwellen 3 nicht hörbar sind, kann die Ermittlung der Körperfunktion parallel zum Abspielen von Musik oder Ähnlichem erfolgen.

### Bezugszeichenliste

- 1: Kopfhörer
- 2: Schallwandler / MEMS-Schallwandler
- 3: Schallwellen / Ultraschallwellen
- 4: Steuereinheit
- 5: Träger
- 6: Ohr
- 7: Gehörgang
- 8: Trommelfell
- 9: reflektierte Ultraschallwellen
- 10: Breitbandspektrum
- 11: Frequenz
- 12: Schalldruckpegel
- 13: Piezoelement
- 14: Membran
- 15: Koppelelement
- 16: Verstärkungsplatte
- 17: Trägereinheit
- 18: Federelement
- 19: Signalgenerator
- 20: Modulator
- 21: Messsignal

## Patentansprüche

1. Kopfhörer (1), insbesondere On-Ear-Hörer, Over-Ear-Hörer und/oder In-Ear-Hörer,
mit zumindest einem Schallwandler (2) zum Erzeugen und Erfassen von zumindest Ultraschallwellen (3), und
mit einer Steuereinheit (4), die anhand der erzeugten und erfassten Ultraschallwellen (3) zumindest ein Merkmal, insbesondere eine Körperfunktion, vorzugsweise eine Herzfunktion, eine Atemfunktion, und/oder eine Identität, eines Trägers (5) des Kopfhörers (1) ermitteln und/oder eine Authentifikation des Trägers (5) durchführen kann,
**dadurch gekennzeichnet,**
**dass** der zumindest eine Schallwandler (2) ein MEMS-Schallwandler (2) ist, der zumindest Ultraschallwellen (3) in einem Breitbandspektrum (10) erzeugen kann.

2. Kopfhörer nach dem vorherigen Anspruch, **dadurch gekennzeichnet, dass** der MEMS-Schallwandler (2) das Breitbandspektrum (10) mit einem hörbaren Wellenlängenspektrum und einem Ultraschallspektrum erzeugen kann, so dass als Schallwellen (3) hörbare Schallwellen (3) und Ultraschwellen (3) erzeugt werden können, wobei der MEMS-Schallwandler (2) vorzugsweise Schallwellen (3) mit dem Breitbandspektrum (10) von größer gleich 3 kHz, 4 kHz oder 15 kHz bis kleiner gleich 40 kHz, 80 kHz oder 100 kHz erzeugen kann.

3. Kopfhörer nach einem oder mehreren der vorherigen Ansprüche, **dadurch gekennzeichnet, dass** die Steuereinheit (4) und/oder der MEMS-Schallwandler (2) derart ausgebildet und/oder eingerichtet sind, dass zur Ermittlung der Körperfunktion ein Messsignal (21) aus Ultraschallwellen (3) mit zumindest einer Messfrequenz aus dem Breitbandspektrum (10) erzeugbar ist, und/oder dass die Steuereinheit (4) und/oder der MEMS-Schallwandler (2) derart ausgebildet und/oder eingerichtet sind, dass die Ultraschwellen (3) des Messsignals (21) ein oder mehrere, insbesondere diskrete, Messfrequenzen umfassen, so dass das Messsignal (21) im Breitbandspektrum (10) einen oder mehrere Messfrequenzpeaks aufweist, und/oder
dass die Ultraschallwellen (3) des Messsignals (21) kontinuierliche Messfrequenzen umfassen, so dass das Messsignal (21) im Breitbandspektrum (10) ein breites Messfrequenzspektrum aufweist.

4. Kopfhörer nach einem oder mehreren der vorherigen Ansprüche, **dadurch gekennzeichnet, dass** die Steuereinheit (4) und/oder der MEMS-Schallwandler (2) derart ausgebildet und/oder eingerichtet sind, dass der zumindest eine Messfrequenzpeak des Messsignals (21) eine Breite von weniger als 3 kHz, 2 kHz oder 1 kHz aufweist, und/oder
dass die Steuereinheit (4) und/oder der MEMS-Schallwandler (2) derart ausgebildet und/oder eingerichtet sind, dass das Messfrequenzspektrum des Messsignals (21) eine Frequenzbreite von größer gleich 1 kHz oder größer gleich 5 kHz oder größer gleich 10 kHz oder größer gleich 20 kHz oder größer gleich 30 kHz oder größer gleich 40 kHz oder größer gleich 50 kHz oder größer gleich 60 kHz oder größer gleich 70 kHz oder größer gleich 80 kHz und/oder von kleiner als 70 kHz oder von kleiner als 60 kHz oder von kleiner als 50 kHz oder von kleiner als 40 kHz oder von kleiner als 30 kHz oder von kleiner als 20 kHz oder von kleiner als 10 kHz aufweist, und/oder
dass der MEMS-Schallwandler (2) derart ausgebildet und/oder eingerichtet ist, dass die im Breitbandspektrum (10) erzeugbaren Schallwellen (3) einen Schalldruckpegel (12) von größer gleich 50 dB, bevorzugt größer gleich 55 dB, besonders bevorzugt größer gleich 70 dB aufweisen, und/oder
dass der MEMS-Schallwandler (2) derart ausgebildet und/oder eingerichtet ist, dass die im Breitbandspektrum (10) erzeugbaren Schallwellen (3) einen Schalldruckpegel (12) von kleiner gleich 90 dB, bevorzugt kleiner gleich 85 dB, besonders bevorzugt kleiner gleich 80 dB, aufweisen, und/oder
dass der MEMS-Schallwandler (2) derart ausgebildet und/oder eingerichtet ist, dass die im Breitbandspektrum (10) erzeugbaren Schallwellen (3) einen Schalldruckpegel (12), insbesondere über das gesamte Breitbandspektrum (10), mit einer Abweichung von +/- 10 dB, insbesondere +/- 5 dB, um einen Mittelwert aufweisen.

5. Kopfhörer nach einem oder mehreren der vorherigen Ansprüche, **dadurch gekennzeichnet, dass** der MEMS-Schallwandler (2) derart ausgebildet und/oder eingerichtet ist, dass die im Breitbandspektrum (10) erzeugbaren Schallwellen (3), insbesondere über das gesamte Breitbandspektrum (10), ein kontinuierliches Spektrum aufweisen.

6. Kopfhörer nach einem oder mehreren der vorherigen Ansprüche, **dadurch gekennzeichnet, dass** der Kopfhörer (1), insbesondere der Signalgenerator (19), einen MLS-Signalerzeuger umfasst, mittels dem ein Maximum-Length-Sequence-Signal erzeugt werden kann, auf Basis dessen der MEMS-Schallwandler (2) zumindest die Ultraschallwellen (3) im Breitbandspektrum (10) erzeugen kann, und/oder
dass der Kopfhörer (1) und/oder die Steuereinheit (4) einen Signalgenerator (19) umfasst, der zeitdiskrete Pulse, Chirp-, Sweep- und/oder Multiton-Signale zum Erzeugen der Ultraschallwellen (3) zum Ermitteln der Körperfunktion erzeugen kann.

7. Kopfhörer nach einem oder mehreren der vorherigen Ansprüche, **dadurch gekennzeichnet, dass** der Kopfhörer (1) und/oder die Steuereinheit (4) einen Modulator (20) umfasst, mittels dem ein zu den hörbaren Schallwellen (3) korrespondierendes elektrisches Signal und ein zum Messsignal (21) im Ultraschallbereich korrespondierendes elektrisches Signal zusammengefasst und/oder moduliert werden kann, wobei der MEMS-Schallwandler (2) aus diesem modulierten Signal die Schallwellen (3) im hörbaren Frequenzbereich und im Ultraschallbereich erzeugen kann, und/oder
dass der MEMS-Schallwandler (2) zumindest ein Piezoelement (13) und eine mit dem zumindest einen Piezoelement (13) gekoppelte Membran (14) umfasst, wobei das zumindest eine Piezoelement (13) und die Membran (14) mittels einer Verstärkungsplatte (16) miteinander gekoppelt sind, so dass die Membran (14) zum Erzeugen der Ultraschallwellen (3) flächig ausgelenkt werden kann.

8. Kopfhörer nach einem oder mehreren der vorherigen Ansprüche, **dadurch gekennzeichnet, dass** mit demselben MEMS-Schallwandler (2) die hörbaren Schallwellen (3) und die Ultraschallwellen (3) erzeugt werden und/oder dass mit demselben MEMS-Schallwandler (2) die Ultraschallwellen (3) erzeugt und erfasst werden können und/oder
dass der Kopfhörer (1) ein Mikrofon und/oder einen weiteren MEMS-Schallwandler (2) umfasst, mittels dem die Ultraschallwellen (3) und/oder vom anderen MEMS-Schallwandler (2) erzeugten Ultraschallwellen (3) erfasst werden können.

9. Verfahren zum Ermitteln zumindest eines Merkmals, insbesondere einer Körperfunktion, vorzugsweise einer Herzfunktion und/oder Atemfunktion, und/oder einer Identität, und/oder zum Durchführen einer Authentifikation eines Trägers (5) eines Kopfhörers (1), insbesondere eines On-Ear-Hörers, Over-Ear-Hörers und/oder In-Ear-Hörers,
bei dem mittels eines MEMS-Schallwandlers (2) des Kopfhörers (1) zumindest Ultraschallwellen (3) aus einem Breitbandspektrum (10) erzeugt werden,
bei dem mittels des MEMS-Schallwandlers (2) und/oder eines weiteren Mikrofons die Ultraschallwellen (3) erfasst werden und
bei dem mittels einer Steuereinheit (4) des Kopfhörers (1) anhand der erzeugten und erfassten Ultraschallwellen (3) das zumindest eine Merkmal des Trägers (5) des Kopfhörers (1) ermittelt wird.

10. Verfahren nach dem vorherigen Anspruch, **dadurch gekennzeichnet, dass** mittels des MEMS-Schallwandlers (2) ein Messsignal (21) mit einer oder mehreren Messfrequenzen aus dem mittels des MEMS-Schallwandlers (2) erzeugbaren Breitbandspektrum (10) zum Ermitteln der Körperfunktion ausgesendet wird und/oder
dass ein Messsignal (21) erzeugt wird, dessen Ultraschallwellen (3) einen oder mehrere Messfrequenzpeaks in einem Breitbandspektrum (10) aufweisen, und/oder
dass ein Messsignal (21) erzeugt wird, dessen Ultraschallwellen (3) ein, insbesondere kontinuierliches, Messfrequenzspektrum im Breitbandspektrum (10) aufweisen.

11. Verfahren nach einem oder mehreren der vorherigen Ansprüche, **dadurch gekennzeichnet, dass** die vom MEMS-Schallwandler (2) erzeugten Ultraschallwellen (3) mit Hilfe eines Maximum-Length-Sequence-Signals erzeugt werden und/oder
dass Ultraschallwellen (3) erzeugt werden, die im Messfrequenzspektrum ein kontinuierliches Spektrum aufweisen und/oder
dass die Messsignale (21) in zeitlichen Abständen zwischen 30 µs und 300 ms, insbesondere zwischen 1 µs und 100 ms, ausgesendet werden.

12. Verfahren nach einem oder mehreren der vorherigen Ansprüche, **dadurch gekennzeichnet, dass** von der Steuereinheit (4) ein Suchprogramm und/oder Suchalgorithmus ausgeführt wird, mittels dem eine zu einem Träger (5) des Kopfhörers (1) passende Messfrequenz des Messsignals (21) ermittelt wird.

13. Verfahren nach einem oder mehreren der vorherigen Ansprüche, **dadurch gekennzeichnet, dass** die Ultraschallwellen (3) erzeugt werden, deren zumindest ein Messfrequenzpeak des Messsignals (21) eine Breite von kleiner als 3 kHz, 2 kHz oder 1 kHz aufweist, und/oder
dass Ultraschallwellen (3) erzeugt werden, deren Messfrequenzspektrum eine Frequenzbreite von größer als 5 kHz, 10 kHz, 20 kHz, 30 kHz, 40 kHz, 50 kHz, 60 kHz, 70 kHz oder 80 kHz und/oder von kleiner als 80 kHz oder von kleiner als 70 kHz oder von kleiner als 60 kHz oder von kleiner als 50 kHz oder von kleiner als 40 kHz oder von kleiner als 30 kHz oder von kleiner als 20 kHz oder von kleiner als 10 kHz oder von kleiner als 5 kHz aufweist, und/oder
dass Ultraschallwellen (3) erzeugt werden, die einen Schalldruckpegel (12) von größer gleich 50 dB, bevorzugt größer gleich 55 dB, besonders bevorzugt größer gleich 70 dB aufweisen, und/oder
dass Ultraschallwellen (3) erzeugt werden, die einen Schalldruckpegel (12) von kleiner gleich 90 dB, bevorzugt kleiner gleich 85 dB, besonders bevorzugt kleiner gleich 80 dB, aufweisen.

14. Verfahren nach einem oder mehreren der vorherigen Ansprüche, **dadurch gekennzeichnet, dass** zum Ermitteln der Körperfunktion mit demselben MEMS-Schallwandler (2) die Ultraschallwellen (3) erzeugt und erfasst werden und/oder dass mittels, insbesondere demselben, MEMS-Schallwandlers (2) die hörbaren Schallwellen (3) und die Ultraschallwellen (3) erzeugt werden, wobei die hörbaren Schallwellen (3) und die Ultraschallwellen (3) vorzugsweise gleichzeitig und/oder simultan erzeugt werden und/oder
dass die Körperfunktion mittels eines Algorithmus und/oder eines Auswertprogramms ausgewertet wird, wobei vorzugsweise die Steuereinheit (4) den Algorithmus und/oder das Auswertprogramm ausführt.

15. Verwendung eines, insbesondere breitbandigen, MEMS-Schallwandlers (2) für einen Kopfhörer (1) und/oder ein Verfahren zum Ermitteln einer Körperfunktion gemäß einem oder mehreren der vorherigen Ansprüche, wobei der MEMS-Schallwandler (2) vorzugsweise zumindest ein in den vorangegangenen Ansprüchen genanntes und den MEMS-Schallwandler (2) betreffendes Merkmal aufweist.
